# FASCICULE DE BREVET EUROPEEN

(11) **EP 0 972 051 B1**
(45) Date de publication et mention de la délivrance du brevet: **11.04.2007**
(21) Numéro de dépôt: 98917263.0
(22) Date de dépôt: 27.03.1998
(51) Int. Cl.: C12N 15/54, C12N 9/12, C12Q 1/68

(54) **ARN-POLYMERASE ARN-DEPENDANTE FONCTIONNANT PREFERENTIELLEMENT SUR MATRICE D'ARN ET PROCEDE DE TRANSCRIPTION D'ARN SOUS LA DEPENDANCE D'UN PROMOTEUR AVEC LEDIT ARN POLYMERASE ARN-DEPENDANTE**
RNS-ABHÄNGIGE RNS-POLYMERASE, DIE VORZUGSWEISE ANHAND EINER RNA-MATRIX FUNKTIONIERT UND EIN PROMOTER-ABHÄNGIGER TRANSKRIPTIONSPROZESS , DER DIESE RNS-ABHÄNGIGE RNS-POLYMERASE BENUTZT.
RNA-DEPENDENT RNA POLYMERASE FUNCTIONING PREFERABLY ON RNA MATRIX AND PROMOTER-DEPENDENT TRANSCRIPTION PROCESS WITH SAID RNA-DEPENDENT RNA POLYMERASE

(30) Priorité: 04.04.1997 FR 9704166
(43) Date de publication de la demande: 19.01.2000
(73) Titulaire: BIO MERIEUX, 69280 Marcy l'Etoile (FR)
(72) Inventeur: CHEYNET-SAUVION, Valérie, F-42410 Verin (FR); ARNAUD-BARBE, Nadège, 69440 Saint Sorlin (FR); ORIOL, Guy, F-42400 Saint Chamond (FR); McALLISTER, William, Edison, NJ 08817 (US); MANDRAND, Bernard, F-69100 Villeurbanne (FR); MALLET, François, F-69100 Villeurbanne (FR)
(74) Mandataire: Le Coupanec, Pascale A.M.P.
(86) Numéro de dépôt international: PCT/FR1998/000635
(87) Numéro de publication internationale: WO 1998/045449

(56) Documents cités:
- EP-A- 0 659 881
- EP-A- 0 747 488
- WO-A-90/06376
- WO-A-95/03426
- WO-A-97/12033
- SOUSA R ET AL: "A mutant T7 RNA polymerase as a DNA polymerase." EMBO (EUROPEAN MOLECULAR BIOLOGY ORGANIZATION) JOURNAL 14 (18). 1995. 4609-4621. ISSN: 0261-4189, XP002049775
- W.T. MCALLISTER: "Structure and function of the bacteriophage T7 RNA polymerase (or, the virtues of simplicity)" CELLULAR AND MOLECULAR BIOLOGY RESEARCH, vol. 39, 1993, pages 385-391, XP002049776
- C.A. RASKIN ET AL.: "Substitution of a single bacteriophage T3 residue in Bacteriophage T7 RNA polymerase at position 748 results in a switch in promoter specificity." JOURNAL OF MOLECULAR BIOLOGY, vol. 228, no. 2, 1992, pages 506-515, XP002049777 cité dans la demande
- C. CAZENAVE ET AL.: "RNA template-directed RNA synthesis by T7 RNA polymerase" PROC. NATL. ACD. SCI. USA, vol. 91, juillet 1994, pages 6972-6976, XP002049779 cité dans la demande
- R. SOUSA: "Structural and mechanistic relationships between nucleic acid polymerases" TIBS, vol. 21, 1996, pages 186-190, XP004050909 cité dans la demande
- S.J. STAHL ET AL.: "Nucleotide sequence of the cloned gene for Bacteriophage T7 RNA polymerase" JOURNAL OF MOLECULAR BIOLOGY, vol. 148, no. 4, 5 juin 1981, pages 481-485, XP002049780 cité dans la demande
- B. VAN GEMEN ET AL.: "Qualitative and quantitative detection of HIV-1 RNA by nucleic acid sequence-based amplification" AIDS, vol. 7, no. 2, novembre 1993, pages s107-s110, XP002073546
- LEARY S.L.; BAUM H.J.; LOEWY Z.G.: 'DNA-dependent RNA polymerase from bacteriophage T3 transcribes and amplifies an RNA template in vitro' GENE vol. 106, no. 1, 30 Septembre 1991, NETHERLANDS, pages 93 - 96, XP000919219 ISSN: 0378-1119
- KONARSKA M.M.; SHARP P.A.: 'REPLICATION OF RNA BY THE DNA-DEPENDENT RNA POLYMERASE OF PHAGE T7' CELL vol. 57, no. 3, 05 Mai 1989, UNITED STATES, pages 423 - 431, XP007901016

## Description

L'invention a pour objet un procédé de transcription, permettant de synthétiser des brins d'ARN complémentaires d'une matrice d'ARN, ainsi que de nouvelles ARN polymérases permettant de mettre en oeuvre ce procédé.

Le procédé de l'invention conduit à l'amplification d'ARN présent en faibles quantités dans un échantillon biologique, et permet ainsi la détection et/ou la quantification de l'ARN de l'échantillon, ou le séquençage du produit de l'amplification, notamment dans le domaine de la microbiologie et de la virologie, et plus généralement dans le domaine du diagnostic médical. Le procédé de l'invention peut également être utilisé dans la synthèse de sondes d'ARN.

On sait qu'en microbiologie et en virologie, les micro-organismes recherchés sont souvent des bactéries viables (et contenant donc davantage d'ARN que d'ADN) ou des virus à ARN comme les virus HIV et HCV. On sait également que dans diverses pathologies, il peut être intéressant de suivre les variations de l'expression des gènes, et donc de la synthèse d'ARN messager.

Il est donc important de pouvoir disposer d'un procédé simple et efficace d'amplification d'une cible d'ARN.

La méthode PCR, qui permet d'amplifier cycliquement une cible d'ADN, utilise une seule enzyme mais nécessite la réalisation de cycles de températures, généralement à trois températures différentes. La méthode PCR peut être adaptée à l'amplification d'une cible d'ARN en ajoutant une activité enzymatique supplémentaire d'ADN polymérase ARN dépendante, ce qui complique encore davantage cette méthode.

La méthode d'amplification dite NASBA/TMA présente l'avantage d'être une méthode isotherme, mais nécessite la mise en oeuvre de trois activités enzymatiques (ADN polymérase ARN dépendante, RNase H, et ARN polymérase ADN dépendante) portées par deux ou trois enzymes.

Il est donc souhaitable de pouvoir disposer d'une méthode simple et automatisable d'amplification d'ARN, et en particulier d'une méthode isotherme n'utilisant qu'une seule enzyme.

Pour éviter les inconvénients, qui viennent d'être évoqués, des techniques d'amplification connues, il apparaît donc nécessaire d'utiliser, pour l'amplification d'ARN, une activité d'ARN polymérase ARN dépendante.

Malheureusement, les ARN polymérases ARN dépendantes (ARNp ARNd) naturelles connues ne sont pas adaptées à une telle utilisation, car elles ont des exigences spécifiques en ce qui concerne la matrice d'ARN, et leur activité nécessite la présence de co-facteurs protéiques (dits aussi facteurs protéiques auxiliaires ou facteurs protéiques associés).

On a maintenant découvert que certaines ARN polymérases ADN-dépendantes connues sont capables de transcrire un ARN simple brin en présence d'un promoteur d'ADN bicaténaire. De plus, certaines de ces enzymes, transformées par mutation, sont capables de synthétiser un produit de transcription avec un meilleur rendement lorsque la matrice est constituée d'ARN que lorsque la matrice est constituée d'ADN.

Dans la présente demande, le terme "transcription" désigne la synthèse de plusieurs brins d'ARN en présence d'une matrice polynucléotidique et de ribonucléosides triphosphates, dans un milieu réactionnel approprié et dans des conditions permettant à l'activité catalytique d'une ARN polymérase de s'exercer. La transcription s'effectue par synthèse d'une copie, complémentaire et anti-parallèle, de la matrice. Le brin de la matrice qui est copié est appelé brin transcrit ou brin matrice. La synthèse de l'ARN progresse dans le sens 5' - 3'.

On sait que certaines ARN polymérases fonctionnent sous la dépendance d'un promoteur. Un promoteur est une séquence nucléotidique double brin reconnue pour l'ARN polymérase et nécessaire à l'initiation de la transcription.

On rappelle que lorsque le brin matrice est lié au promoteur, le premier nucléotide transcrit sur le brin matrice, lié par son extrémité 3' à l'extrémité 5' de l'un des brins du promoteur, est désigné par +1. Le brin du promoteur qui est lié au brin matrice est appelé brin anti-sens. L'autre brin du promoteur, complémentaire du brin anti-sens, et hybridé à celui-ci, est appelé brin sens. Les nucléotides successifs qui sont situés du côté du promoteur, par rapport au nucléotide +1, sont, en partant de +1, numérotés -1, -2, -3, etc.

La position - 1 correspond donc à l'extrémité 5' du brin anti-sens du promoteur, et à l'extrémité 3' du brin sens. Cependant, certains auteurs incluent la séquence nucléotidique correspondant à la région où débute la transcription (notamment la séquence de +1 à +6, pour laquelle on peut généralement définir une séquence consensus) dans la définition de la séquence du promoteur.

Sur le brin matrice, les positions des nucléotides successifs copiés, à partir de +1, et donc dans la direction 3' - 5', sont notées +2, +3, etc ...

Dans ce qui suit, on parle généralement de brin sens et de brin anti-sens pour le promoteur proprement dit (positions numérotées négativement), et on parle de brin non-matrice pour tout brin lié à l'extrémité 3' du brin sens, et de brin matrice pour tout brin lié à l'extrémité 5' du brin anti-sens ou pour tout brin hybridé au brin non-matrice. Dans un brin polynucléotidique donné, on appelle "région amont" une région située du côté de l'extrémité 5', et "région aval" une région située du côté de l'extrémité 3'. Cependant, dans le domaine de la transcription sous la dépendance d'un promoteur, et sans prendre en considération un brin particulier, on appelle traditionnellement région "amont" la région qui, par rapport à la position +1, se trouve du côté du promoteur (positions indiquées par des nombres négatifs), et région "aval" la région située du côté de la matrice copiée (positions indiquées par des nombres positifs), de sorte que la direction aval correspond alors au sens 3'- 5' sur le brin matrice, et au sens 5'- 3' sur le brin d'ARN néosynthétisé.

Le brin matrice n'est pas nécessairement lié à l'extrémité 5' du brin anti-sens du promoteur. Mais il doit être dans ce cas hybridé à un brin complémentaire et anti-parallèle (brin non-matrice) lui-même lié par son extrémité 5' à l'extrémité 3' du brin sens du promoteur ; voir ZHOU W. et DOETSCH P.W., *Biochemistry* **33,** 14926-14934 (1994) et ZHOU W. *et al.*, *Cell* **82,** 577-585 (1995). Dans un tel cas, la transcription peut débuter en toute position , pouvant aller de +1 à + 24 , correspondant à l'extrémité 3' du brin matrice ou de la partie du brin matrice hybridée sur le brin non matrice.

Comparées aux ARN polymérases bactériennes, eucaryotes ou mitochondriales, les ARN polymérases phagiques sont des enzymes très simples. Parmi celles-ci, les mieux connues sont les ARN polymérases des bactériophages T7, T3 et SP6. L'ARN polymérase du bactériophage a été clonée ; voir notamment le brevet US 4 952 496. Ces enzymes sont très homologues entre elles et sont formées par une seule sous-unité. Les promoteurs naturels spécifiques des ARN polymérases des phages T7, T3 et SP6 sont bien connus. Le séquençage du génome entier du bactériophage T7 (Dunn *et al., J. Mol. Biol.* **166**, 477-535 (1983)) a permis de définir l'existence de 17 promoteurs sur l'ADN de ce phage. La comparaison de ces 17 séquences montre que 23 nucléotides contigus situés entre les positions - 17 et + 6 par rapport au site d'initiation (position +1) de la transcription, sont fortement conservés. Ces nucléotides sont même identiques chez cinq promoteurs dits de classe III, qui sont les plus efficaces notamment *in vitro.* De même, de nombreuses séquences promotrices spécifiques pour la T3 ARN polymérase présentent également une homologie très importante, notamment entre les positions - 17 et + 6. Par ailleurs, plusieurs séquences différentes de promoteur pour l'ARN polymérase du phage SP6 ont été mises en évidence et présentent également une forte homologie ; voir Brown J.E., *et al., Nucleic Acids Res*. **14**, 3521-3526 (1986).

Il est donc possible de considérer que les diverses ARN polymérases phagiques mentionnées ci-dessus font partie d'une famille d'ARN polymérases qui reconnaissent des promoteurs présentant une séquence consensus de la position - 17 à la position + 6 , et notamment de la position - 17 à la position - 1.

Le procédé de l'invention permet de transcrire une séquence quelconque d'ARN, car les ARN polymérases, capables de transcrire de l'ARN sous la dépendance d'un promoteur, qui sont décrites dans la présente demande , peuvent transcrire l'ARN sans spécificité importante de séquence. On sait toutefois que certaines séquences de début de transcription, notamment de la position + 1 à la position +6, sont plus favorables que d'autres pour obtenir des transcrits de longueur attendue avec une ARN polymérase phagique donnée, dans le cas de la transcription d'ADN ; voir par exemple Milligan J. F. *et al., Nucleic Acids Research,* **15**, 8783-8798 (1987). Les ARN polymérases capables de transcrire de l'ARN qui sont décrites dans la présente demande peuvent elles aussi fonctionner avec des rendements variables selon la séquence de la région de début de transcription. Les séquences qui conviennent le mieux à une ARN polymérase donnée peuvent être déterminées, le cas échéant, par de simples expériences de routine analogues à celles décrites par Milligan *et al.* dans l'article qui vient d'être mentionné. En outre, comme on le verra ci-après, le procédé de trancription de l'invention permet, le cas échéant, soit de faire débuter la transcription dans une région favorable de l'ARN à transcrire, soit de fournir un réactif-promoteur qui contient déjà une région de début de transcription ayant une séquence favorable pour une ARN polymérase donnée.

La présente invention a donc pour objet un procédé d'amplification d'une séquence cible quelconque d'ARN, par transcription sous la dépendance d'un promoteur, dans un échantillon d'ARN comprenant ladite séquence cible,
dans lequel on met en contact ledit échantillon :
- avec un réactif capable de s'hybrider avec ledit ARN comprenant ladite séquence cible,
- et avec un système enzymatique comprenant une activité d'ARN polymérase ARN-dépendante,
dans des conditions permettant l'hybridation dudit réactif avec ledit ARN comprenant ladite séquence cible et dans des conditions permettant le fonctionnement de ladite activité d'ARN polymérase ARN-dépendante ;
dans lequel ledit réactif contient :
(i) un premier brin nucléotidique comprenant : a) un premier segment nucléotidique capable de jouer le rôle de brin sens d'un promoteur pour ladite activité d'ARN polymérase et b), en aval dudit premier segment, un second segment nucléotidique comprenant une séquence capable d'hybridation avec une région dudit ARN, et
(ii) à l'état hybridé sur le premier brin, un second brin nucléotidique comprenant un troisième segment nucléotidique capable d'hybridation avec ledit premier segment de façon à former avec lui un promoteur double brin fonctionnel ;
et dans lequel ladite activité d'ARN polymérase est capable de transcrire une matrice d'ARN, en présence dudit réactif hybridé sur ladite matrice, en l'absence de facteur protéique associé et en l'absence d'une activité de ligase.

Les conditions générales permettant l'hybridation de brins nucléotidiques sont connues, et des conditions particulières peuvent être facilement déterminées, par des expériences de routine, pour des brins de séquence donnée. Les conditions permettant le fonctionnement de l'activité d'ARN polymérase, en présence de ribonucléosides triphosphates, peuvent être elles aussi facilement déterminées parl'expérience, éventuellement avec l'aide des indications fournies dans la partie expérimentale ci-après.

L'extrémité 3' du premier segment correspond à la position -1 dans le système transcriptionnel utilisé. Le premier segment contient un nombre suffisant de nucléotides pour pouvoir, à l'état hybridé, jouer le rôle d'un promoteur pour une ARN polymérase. Selon un mode de réalisation particulier, le premier segment contient au moins 9 nucléotides.

Dans le brevet FR 2 714 062, il a été montré que des séquences courtes de 6 à 9 nucléotides consécutifs choisis dans la région -12 à -4 du brin sens d'un promoteur pour une ARN polymérase phagique sont capables de jouer le rôle de promoteurs fonctionnels dans la transcription d'une séquence cible d'ADN.

Le réactif utilisé dans le procédé de l'invention peut encore présenter l'une au moins des caractéristiques suivantes :
- ledit troisième segment est flanqué, à son extrémité amont, par un quatrième segment nucléotidique qui est plus court que ledit second segment du premier brin ;
- ledit quatrième segment est capable d'hybridation avec une partie en regard dudit second segment.
- lesdits premier et troisième segments sont constitués d'ADN ;
- lesdits troisième et quatrième segments sont constitués d'ADN ou d'ARN.

Le troisième segment peut avoir la même longueur que le premier segment. Il peut être aussi plus court ou plus long, mais son extrémité 5' doit correspondre à la position -1 (c'est-à-dire la position précédant immédiatement la position de début de transcription dans le cas où le brin matrice est lié au promoteur), lorsqu'il est hybridé sur le premier segment.

Lorsque le second brin du réactif ne contient pas le quatrième segment, le réactif peut être utilisé notamment pour transcrire un ARN dont la région d'extrémité 3', ou une région voisine de l'extrémité 3', a une séquence connue, et dans ce cas le second segment nucléotidique du premier brin est construit de façon que ledit ARN, au voisinage de son extrémité 3', soit capable d'hybridation avec au moins une partie de la séquence dudit second segment nucléotidique. L'extrémité 3' de la partie de l'ARN à transcrire qui est hybridée au second segment peut être contiguë à l'extrémité 5' du troisième segment, ou bien elle peut en être éloignée par un nombre x de nucléotides (comptés sur le second segment), x représentant zéro ou un nombre entier de 1 à 24. Bien entendu, la longueur du second segment (en nombre de nucléotides) est supérieure à x, pour pouvoir assurer la fixation de la matrice ARN à transcrire, par hybridation sur une région aval du second segment.

Le quatrième segment, contenant par exemple de 1 à 18 nucléotides, et en particulier de 1 à 12 nucléotides, a de préférence une séquence choisie pour favoriser le début de la transcription pour une ARN polymérase donnée (voir notamment la partie expérimentale ci-après). Le quatrième segment peut être réalisé notamment en ADN. Sa séquence peut être complémentaire de la région amont du second segment qui lui fait face et à laquelle il est alors hybridé. Dans ce cas, le choix de la séquence de la région 5' du deuxième segment est dicté par le choix de la séquence du quatrième segment. Il n'est pas nécessaire que le quatrième segment soit lié au troisième segment puisque de toute façon son positionnement correct en vue de favoriser le début de la transcription peut être assuré par son hybridation au second segment. Toutefois, dans un mode de réalisation particulier, le quatrième segment est lié au troisième segment.

Comme précédemment, l'extrémité 3' de la partie de l'ARN cible qui est hybridée sur le second segment peut être éloignée de l'extrémité 5' du quatrième segment par un nombre de nucléotides égal à x, tel que défini ci-dessus.

Pour des raisons évidentes, le second segment contient un nombre de nucléotides au moins égal à la somme du nombre de nucléotides du quatrième segment, s'il est présent, et du nombre de nucléotides de ladite séquence du second segment qui est capable d'hybridation avec ladite région de l'ARN à transcrire.

Le procédé de transcription de l'invention peut être mis en oeuvre avec une ARN polymérase de type sauvage de virus ou de phage, et en particulier avec une ARN polymérase choisie dans la famille des ARN polymérases, mentionnée ci-dessus, qui inclut la T7 ARN polymérase, la T3 ARN polymérase et la SP6 ARN polymérase.

On a en effet découvert que ces ARN polymérases, connues pour être ADN-dépendantes, étaient également capables de transcrire une matrice d'ARN, éventuellement en choisissant (par exemple grâce au quatrième segment décrit ci-dessus), une séquence favorisant le début de la transcription.

La découverte de cette activité d'ARN polymérase ARN-dépendante permet de disposer pour la première fois d'ARN polymérases capables de transcrire de l'ARN sous la dépendance d'un promoteur, dès la position +1, et en l'absence de facteur protéique associé.

On peut également mettre en oeuvre le procédé de l'invention avec des ARN polymérases mutées qui seront décrites plus en détail ci-après . L'intérêt de ces ARN polymérases mutées est que certaines d'entre elles sont capables d'effectuer la transcription avec un meilleur rendement lorsque la matrice est constituée d'ARN que lorsque la matrice est constituée d'un ADN comparable (c'est-à-dire contenant les désoxyribonucléotides A, C, G à la place des ribonucléotides A, C, G, respectivement, et contenant le désoxyribonucléotide T à la place du ribonucléotide U).

L'invention concerne également l'utilisation d'une ARN polymérase capable de transcrire une matrice d'ARN, sous la dépendance d'un promoteur, en l'absence de facteur protéique auxiliaire, dans un procédé de transcription d'un brin matrice comprenant une séquence cible d'ARN dans laquelle ladite ARN polymérase est choisie parmi la T7 ARN, la SP6 ARN polymérase et les ARN polymérases mutées telles que définies ci-dessus. Le brin matrice peut être constitué d'ARN, ou encore être constitué d'ADN dans la région de début de transcription, puis d'ARN ensuite.

L'invention concerne aussi l'utilisation d'une ARN polymérase capable de transcrire une matrice d'ARN, sous la dépendance d'un promoteur, en l'absence de facteur protéique auxiliaire, dans un procédé de transcription d'un brin matrice comprenant une séquence cible d'ARN, dans laquelle ledit brin matrice est constitué d'ARN au moins entre la position +5 et l'extrémité 5' de la cible ladite ARN polymérase étant une ARN polymérase sauvage de virus ou de phage. Le brin matrice est donc constitué d'ARN à partir d'une des positions +1 à +5, et peut donc être constitué d'ADN de la position +1 à la position +2, +3 ou +4. L'article de S. Leary et *al*. mentionné ci-après décrit la transcription d'une matrice constituée d'ADN pour les positions +1 à +6 puis d'ARN pour les positions 7 et suivantes, avec la T3 ARN polymérase.

L'invention concerne également des ARN polymérases ARN-dépendantes (ARNpARNd), obtenues par modification d'ARN polymérases ADN-dépendantes, et qui sont capables de synthétiser des brins d'ARN complémentaires d'une matrice d'ARN. Elles peuvent être utilisées par exemple dans le séquençage d'ARN, la synthèse de sondes d'ARN, et les techniques d'amplification permettant en particulier la détection et la quantification d'ARN.

Les ARNpARNd naturelles connues ne sont pas adaptées pour servir de polymérases dans plusieurs applications, parce qu'elles ont acquis une forte capacité discriminatoire vis-à-vis de leur ARN matrice spécifique. De plus, ces enzymes ne sont pas bien caractérisées. Pour la plupart, elles forment des complexes supra-moléculaires composés à la fois de facteurs viraux et cellulaires ; ces complexes, qui sont généralement associés aux membranes, sont difficiles à purifier et sont instables au cours de l'isolement (B. N. Fields, D. M. Knipe, *Virology. Vols 1 and 2,* Raven Press, New York, (1990) ; G. P. Pfeifer, R. Drouin, G. P. Holmquist, *Mutat. Res. Fundam. Mol. Mech. Mutagen.* **288**, 39 (1993)).

Peu d'ARNpARNd ont été clonées, séquencées et exprimées. L'enzyme Qβ réplicase est la mieux caractérisée. Cette enzyme est composée de 4 sous-unités, dont 3 sont des facteurs de l'hôte (M. Kajitani, A. Ishihama, *Nucleic Acids Res.* **19**, 1063 (1991)). L'enzyme Qβ a été isolée ; elle montre une bonne processivité et est capable de réaliser des réactions cycliques (P. M. Lizardi, C. E. Guerra, H. Lomeli, I. Tussie-Luna, F. R. Kramer, *Bio*/*Technology* **6**, 1197 (1988)). Cependant, cette enzyme reste très limitée dans ses applications car elle ne reconnaît comme matrice qu'une classe restreinte de molécules d'ARN fortement structurées (V. D. Axelrod, E. Brown, C. Priano, D. R. Mills, *Virology* **184**, 595 (1991)).

Une autre ARNpARNd a été partiellement caractérisée. Il s'agit de l'enzyme du virus L-A de *Saccharomyces cerevisiae.* Cette polymérase, qui a été clonée, est nécessaire à l'assemblage de la particule virale ; elle se fixe tout d'abord à l'ARN brin plus, puis induit l'assemblage des protéines de la particule (T. Fujimura, R. Esteban, L. M. Esteban, R. B. Wickner, *Cell* **62**, 819 (1990)). Au moins trois facteurs sont connus pour s'associer avec les particules virales. Ces facteurs sont nécessaires à la réplication de l'ARN, à la transcription, et au maintien cohérent de la particule (T. Fujimura, R. B. Wickner, *Molec. Cell. Biol.* **7**, 420 (1987)). Les études *in vitro* ont montré qu'une particule virale intacte est nécessaire à la synthèse du brin moins (réplication) (T. Fujimura, R. B. Wickner, *Cell* **55**, 663 (1988)) et à la synthèse du brin plus (transcription) (T. Fujimura, R. B. Wickner, *J. Biol. Chem.* **264**, 10872 (1989)). Ainsi, la complexité de ce système ne le rend pas facilement adaptable à un système de transcription *in vitro.* De plus, tout comme le système Qβ, ce système est très discriminatoire, acceptant seulement les ARN viraux L-A et M comme matrice (T. Fujimura, R. B. Wickner, *Cell* **55**, 663 (1988)).

Une ARNpARNd pour laquelle il a été montré une capacité étendue d'acceptation de matrice est l'enzyme du virus de la poliomyélite (J. Plotch, O. Palant, Y. Gluzman, *J. Virol.* **63**, 216 (1989)). Cependant, plusieurs problèmes existent avec ce système enzymatique : l'amorçage est dépendant soit d'un facteur de l'hôte non identifié soit de l'addition d'un oligonucléotide poly(U). L'amorçage par un oligonucléotide poly(U) n'étant cependant pas sélectif vis-à-vis de la matrice, de nombreux produits de différentes tailles sont synthétisés, notamment des produits ayant deux fois la longueur de la matrice. De plus, la synthèse séquentielle des brins plus et moins n'a pas été démontrée (S. J. Plotch, O. Palant, Y. Gluzman, *J. Virol.* **63**, 216 (1989), T. D. Hey, O. C. Richards, E. Ehrenfeld, *J.* Virol. **61**, 802 (1987), J. M. Lubinski, L. J. Ransone, A. Dasgupta, *J*. *Virol.* **61**, 2997 (1987)).

Parmi les ARNpADNd, les enzymes des bactériophages T3 et T7 sont capables d'utiliser l'ARN comme matrice dans des conditions particulières. Par exemple, l'ARNpADNd T3 peut transcrire une matrice ARN simple brin (i.e. l'ARN messager du gène de la résistance à la néomycine) si elle est ligaturée au brin anti-sens du promoteur T3 incluant la séquence d'initiation de + 1 à + 6 (S. Leary., H. J. Baum, Z. G. Loewy, *Gene* **106**, 93 (1991)). Il est également connu que la T7 ARN polymérase peut transcrire, d'une extrémité à l'autre, une matrice ARN en l'absence de la séquence promotrice (M. Chamberlin., J. Ring, *J. Biol. Chem.* **248**, 2235 (1973)). De plus, il a été montré que la T7 ARN polymérase peut efficacement transcrire deux petites matrices ARN spécifiques, les ARN "X" et "Y", produisant à la fois des copies d'ARN plus et moins. Cette réplication, obtenue en l'absence d'une séquence promotrice consensus, semble dépendante de la présence d'une structure secondaire spécifique (M. M. Konarska, P. A. Sharp, *Cell* **57**, 423 (1989), M. M. Konarska, P. A. Sharp, *Cell* **63.**, 609 (1990)). En revanche, les ARN "X" et "Y" ne sont pas répliqués par la T3 ARN polymérase, et on ne sait pas si cette enzyme n'est pas capable de répliquer des ARN hautement structurés, ou si la spécificité de séquence de cette enzyme empêche sa reconnaissance des ARN "X" et "Y". En l'absence de promoteur, il a également été montré que la T7 ARNpADNd était capable d'effectuer l'élongation de deux brins d'ARN chevauchants en anti-sens (C. Cazenave and O.C Ulhlenbeck, *Proc. Natl. Acad. Sci. USA* **91**, 6972 (1994)). De même, il a été montré que la T7 ARNpADNd sauvage est capable d'effectuer l'élongation d'une amorce ARN sur matrice ADN simple brin, en l'absence de promoteur (S.S. Daube and P.H. von Hippel, *Biochemistry* **33**, 340 (1994)).

L'enzyme des bactériophages la mieux caractérisée est la T7 ARN polymérase, une enzyme monomère de 98 kDa (B. A. Moffatt, J. J. Dunn, F. W. Studier, *J. Mol. Biol.* **173,** 265 (1984)). Cette polymérase monomère possède toutes les propriétés essentielles d'une ARN polymérase, c'est-à-dire reconnaissance d'un promoteur, initiation de la transcription, élongation et terminaison (M. Chamberlin, T. Ryan, *The Enzymes* **XV**, 87 (1982)). De plus, l'activité catalytique nécessite peu d'éléments, à savoir une matrice, des ribonucléosides triphosphates et l'ion divalent Mg²⁺, et elle ne nécessite aucun facteur auxiliaire protéique pour initier ou terminer la transcription, contrairement aux autres ARN polymérases (M. Chamberlin, T. Ryan, *The Enzymes* **XV**, 87 (1982)).

La mutagénèse du gène de la T7 ARN polymérase a permis d'identifier et de définir des régions ou des résidus impliqués dans la fonction polymérase. Une stratégie de mutagenèse a consisté à échanger des éléments entre la T7 ARN polymérase et sa proche cousine la T3 ARN polymérase dont la séquence en acides aminés est identique à 82 % (K. E. Joho, L. B. Gross, N. J. McGraw, C. Raskin, W. T. McAllister, *J. Mol. Biol*. **215**, 31 (1990)). Cette stratégie a conduit à l'identification d'éléments de la polymérase impliqués dans la reconnaissance du promoteur. Il a été montré, par exemple, que la substitution d'un seul acide aminé dans l'enzyme T3 (ou T7) permet à l'enzyme mutée de reconnaître spécifiquement le promoteur T7 (ou T3) hétérologue (C. A. Raskin, G. Diaz, K. Joho, W. T. McAllister, *J. Mol. Biol.* **228**, 506 (1992)). De la même façon, des substitutions réciproques dans les séquences promotrices respectives confèrent au promoteur muté la capacité d'être reconnu par l'enzyme hétérologue (C. A. Raskin, G. Diaz, K. Joho, W. T. McAllister, *J. Mol. Biol.* **228**, 506 (1992)).

La T7 ARN polymérase a été cristallisée et sa structure déterminée à une résolution de 3, 3 Å (R. Sousa, Y. J. Chung, J. P. Rose, B. -C. Wang, *Nature* **364**, 593 (1993)). A partir de cette étude structurale, des alignements de séquences (K. E. Joho, L. B. Gross, N. J. McGraw, C. Raskin, W. T. McAllister, *J. Mol. Biol.* **215**, 31 (1990) ; S. Mungal, B. M. Steinberg, L. B. Taichman, *J. Virol.* **66**, 3220 (1992), W. T. McAllister, C. A. Raskin, *Molec. Microbiol.* **10**, 1 (1993)) et des études de mutagenèse (D. Patra, E. M.Lafer, R. Sousa, *J. Mol. Biol.* **224**, 307 (1992) ; L. Gross, W-J. Chen, W. T. McAllister, *J. Mol. Biol.* **228**, 1 (1992)), il a été possible de corréler les éléments fonctionnels de l'enzyme T7 aux éléments structuraux. La T7 ARN polymérase peut être divisée en deux domaines fonctionnels : un domaine de reconnaissance du promoteur et un domaine catalytique (R. Sousa, Y. J. Chung, J. P. Rose, B. -C. Wang, *Nature* **364**, 593 (1993), W. T. McAllister, *Cell. Molec. Biol. Res.* **39**, 385 (1993)).

L'asparagine 748 de la T7 ARN polymérase a été montrée comme interagissant avec les nucléotides -10 et -11 dans la séquence promoteur, une interaction montrée comme responsable de la spécificité de promoteur (C.A. Raskin, G. Diaz, K. Joho, W.T. McAllister, *J. Mol. Biol.* **228**, 506 (1922)). Il a été évoqué la possibilité qu'une interaction de type sigma entre la T7 polymérase et son promoteur puisse exister dans le système du bactériophage. En effet, une séquence de type sigma, correspondant à la région 2.4 de sigma, i.e. la région de sigma interagissant avec la "Pribnow box" (séquence TATAATG reconnue par le facteur de transcription sigma 70 d'*E.coli*) (C. Waldburger, T. Gardella, R. Wong, M.M. Susskind, *J. Mol. Biol.* **215**, 267 (1990) ; D.A. Siegele, J.C. Hu, W.A. Walter, C.A. Gross, *J. Mol. Biol.* **206**, 591 (1989)), existe dans la région N-terminale de la T7 ARN polymérase entre les acides aminés 137 et 157 (L. Gross, W-J. Chen, W.T. McAllister, *J. Mol. Biol.* **228**, 1 (1992)). D'autre part, bien qu'aucune fonction n'ait pu lui être attribuée, la région 230 à 250 présente des homologies de séquence avec le répresseur λ de *E.coli* (McGraw, N.J., Bailey, J.N., Cleaves, G.R., Dembinski, D.R., Gocke, C.R., Joliffe, L.K., MacWright, R.S. and McAllister, W.T. *Nucleic Acids Res.* **13**, 6753 (1985)).

Le domaine catalytique consiste en une poche résultant de la mise à proximité de plusieurs régions dispersées sur la structure primaire (R. Sousa, Y.J. Chung, J.P. Rose, B.-C. Wang, *Nature* **364**, 593 (1993), W.T. McAllister, C.A. Raskin, *Molec. Microbiol.* **10**, 1 (1993) ; D. Moras, *Nature* **364**, 572 (1993)). Cette poche contient notamment plusieurs motifs conservés parmi lesquels les motifs A et C sont les mieux conservés chez les polymérases (Poch, O., Sauvaget, I., Delarue, M. and Tordo, N. EMBO J. **8**, 3867 (1989) ; Delarue, M., Poch, O., Tordo, N. and Moras, D. Protein Engineering **3**, 461 (1990) ; W.T. McAllister, C.A. Raskin, *Molec. Microbiol.* **10**, 1 (1993)). Un troisième motif, le motif B, est conservé chez les ARN et ADN polymérases ADN dépendantes, tandis qu'un motif B' différent (aussi bien pour la séquence que pour la structure apparente) existe chez les ARN et ADN polymérases ARN dépendantes (Poch, O., Sauvaget, I., Delarue, M. and Tordo, N. EMBO J. **8**, 3867 (1989) ; Delarue, M., Poch, O., Tordo, N. and Moras, D. Protein Engineering 3, 461 (1990) ; L.A. Kohlstaedt, J. Wang, J.M. Friedman, P.A. Rice, T.A. Steitz, *Science* **256**, 1783 (1992) ; W.T. McAllister, C.A. Raskin, *Molec. Microbiol.* **10**, 1 (1993)).

SOUSA R ET AL: "A mutant T7 RNA polymerase as a DNA polymerase." EMBO (EUROPEAN MOLECULAR BIOLOGY ORGANIZATION) JOURNAL 14 (18), 1995. 4609-4621 divulgue des ARN polymérases mutées.

L'un des aspects de la présente invention repose sur la découverte que certaines ARN polymérases ADN-dépendantes mutées sont capables de transcrire un ARN simple brin ou double brin en présence d'un promoteur ADN bicaténaire. De plus, ces enzymes mutantes sont peu capables ou incapables de transcrire de l'ADN simple ou double brin en présence d'un promoteur ADN bicaténaire Elles sont donc préférentiellement ou strictement ARN-dépendantes. Leur utilisation est particulièrement intéressante dans les cas où l'on souhaite transcrire sélectivement l'ARN, en particulier lorsque l'échantillon biologique de départ contient ou risque de contenir de l'ADN ayant une séquence identique ou semblable à celle de l'ARN à amplifier.

L'invention a donc pour objet une ARN polymérase capable de transcrire un segment polynucléotidique d'intérêt de séquence quelconque contenu dans une matrice polynucléotidique, en synthétisant, en présence de ladite matrice, et sous la dépendance d'un promoteur, un produit de transcription contenant une séquence d'ARN complémentaire de la séquence dudit segment polynucléotidique d'intérêt, caractérisée par le fait qu'elle est mutée et capable de synthétiser ledit produit de transcription avec un meilleur rendement lorsque ladite séquence d'intérêt contenue dans la matrice est constituée d'ARN que lorsque ladite séquence d'intérêt contenue dans la matrice est constituée d'ADN.

L'invention concerne en particulier une ARN polymérase définie comme ci-dessus telle que le rapport du rendement en produit de transcription d'une matrice ADN au rendement en produit de transcription d'une matrice ARN, exprimé en %, est inférieur à 95 %, notamment inférieur à 85 % et en particulier inférieur à 70 %.

L'invention a notamment pour objet une ARN polymérase telle que définie ci-dessus, caractérisée par le fait que le rapport du rendement en produit de transcription de la matrice ARN au rendement en produit de transcription de la matrice ADN est au moins égal à 2, et en particulier au moins égal à 10.

Le "rendement" de la transcription est le rapport molaire de la quantité de produit de transcription à la quantité de matrice polynucléotidique présente à l'origine. Ce rendement peut être facilement déterminé par l'expérience, en introduisant dans le milieu réactionnel une quantité déterminée de la matrice polynucléotidique. Pour la comparaison des rendements obtenus avec une matrice d'ADN et une matrice d'ARN, il faut évidemment que les conditions autres que celles de la nature de la matrice soient comparables.

L'ARN polymérase de l'invention est capable de transcrire une matrice polyribonucléotidique de séquence quelconque, et elle se distingue en cela de la Qβ-réplicase. Elle transcrit préférentiellement ou exclusivement une matrice d'ARN, et elle se distingue en cela des ARN polymérases ADN-dépendantes de phages connues.

Les ARN polymérases de l'invention, contrairement aux ARNp ARNd naturelles connues, sont notamment des ARN polymérases capables de fonctionner sans cofacteur(s) protéique(s) associé(s). Elles peuvent toutefois se présenter sous la forme de multimères, et en particulier de dimères.

Les ARN polymérases mutées de l'invention sont donc généralement obtenues au départ d'ARN polymérases elles-mêmes capables de fonctionner sans cofacteurs protéiques.

Les ARN polymérases de l'invention peuvent être notamment des ARN polymérases dérivant par mutation d'une ARN polymérase ADN-dépendante de virus ou de phage, et en particulier d'une ADN polymérase d'un phage de E.coli. Parmi les phages de E.coli, on peut citer notamment T3, T7 et SP6.

Une ARN polymérase selon l'invention peut posséder une homologie de séquence protéique supérieure à 50 %, et en particulier supérieure à 80 % avec une ARN polymérase de type sauvage de la famille des ARN polymérases ADN-dépendantes incluant la T7 ARN polymérase, la T3 ARN polymérase et la SP6 ARN polymérase.

La famille d'ARN polymérases ADN-dépendantes mentionnée ci-dessus est connue ; voir par exemple l'article de R. Sousa, TIBS 21, 186-190 (1996), et les références citées dans cet article.

Parmi les polymérases de l'invention on citera en particulier celles qui contiennent au moins une mutation dans une région correspondant à la séquence d'acides aminés 625-652 de la T7 ARN polymérase, et notamment celles qui ont la composition d'une ARN polymérase ADN-dépendante de type sauvage, à l'exception du fait qu'elles comportent au moins une mutation dans ladite région. On entend ici par "mutation" le remplacement, la délétion ou l'insertion d'un acide aminé.

On citera par exemple les ARN polymérases comportant au moins une mutation en une position correspondant à l'une des positions 627, 628, 631, 632 et 639 de la séquence d'acides aminés de la T7 ARN polymérase ; en particulier ladite mutation peut comprendre le remplacement d'un résidu d'acide aminé, choisi parmi l'arginine, la lysine, la sérine et la tyrosine, de l'ARN polymérase de type sauvage par un autre résidu d'acide aminé. L'acide aminé remplacé est par exemple une arginine ou une lysine. L'acide aminé de remplacement peut être notamment choisi parmi l'alanine, la valine, la leucine, l'isoleucine, la glycine, la thréonine ou la sérine. On comprend que l'expression "acide aminé" désigne ici, par abus de langage, un résidu d'acide aminé engagé dans une liaison peptidique.

On a fait référence ci-dessus à la séquence peptidique de la T7 ARN polymérase. La numérotation des résidus d'acides aminés adoptée ici est celle décrite par Dunn, J. J. et Studier, F. W. J. Mol. Biol. 148(4), 303-330 (1981), et par Stahl, S. J. et Zinn, K., J. Mol. Biol. 148(4), 481-485 (1981).

L'invention concerne également:
- un gène codant pour une ARN polymérase telle que définie précédemment ; un tel gène peut être obtenu par exemple selon une méthode analogue à celle décrite ci-après dans la partie expérimentale ;
- un vecteur d'expression dans lequel est inséré un tel gène, ledit vecteur étant capable d'exprimer ladite ARN polymérase dans une cellule-hôte ; ce vecteur peut être obtenu de façon connue en soi :
- une cellule-hôte contenant un tel vecteur.

L'invention concerne également un procédé d'obtention d'une ARN polymérase telle que définie précédemment, caractérisé par le fait : a) que l'on obtient de façon connue un gène codant pour une ARN polymérase de type sauvage, b) que l'on effectue au moins une mutation sur ledit gène, c) que l'on insère le gène muté obtenu dans un vecteur d'expression, d) que l'on fait s'exprimer ledit vecteur dans une cellule-hôte pour obtenir une ARN polymérase mutée et e) que l'on sélectionne parmi les ARN polymérases mutées obtenues celles qui présentent l'une au moins des propriétés d'une ARN polymérase telle que définie ci-dessus.

On va donner ci-après une description plus détaillée d'un mode d'exécution particulier du procédé de l'invention, dans le cas de l'utilisation de la T7 ARN polymérase comme produit de départ.

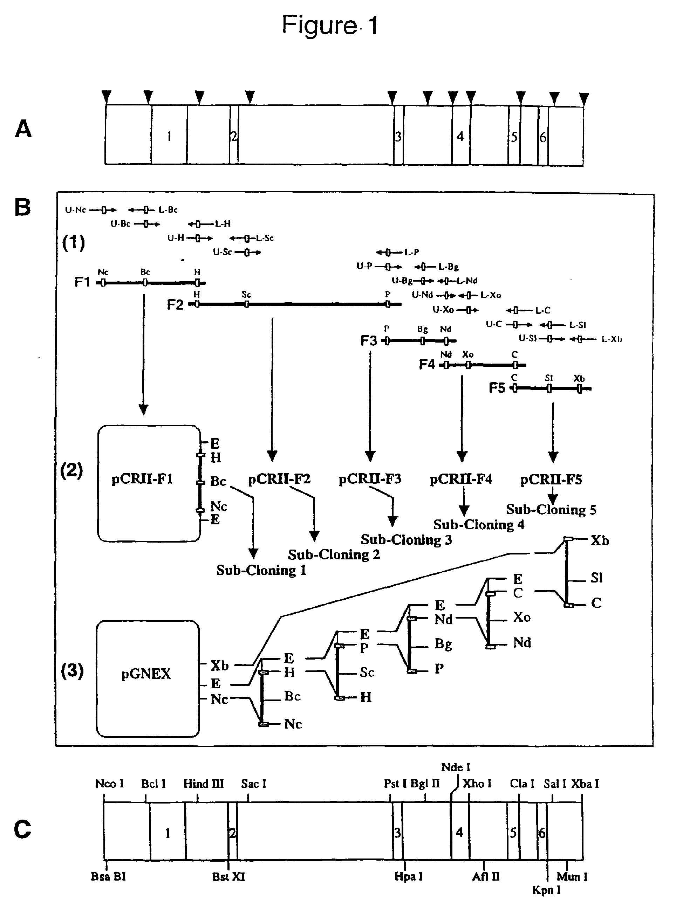

On a préparé un gène modulaire de la T7 ARNpADNd, ce gène résultant de l'assemblage de différentes cassettes (voir l'exemple 1 et la figure 1).

Le gène modulaire ainsi défini est caractérisé par le fait qu'il contient 10 cassettes bordées par des sites de restriction uniques dans le vecteur de clonage.

En particulier, ces cassettes, bordées par des sites de restriction uniques, sont caractérisées par le fait que chaque cassette comprend une région d'intérêt, notamment celles impliquées dans la reconnaissance de promoteur (région présentant une homologie avec le facteur *σ* d'*E.coli* ; région présentant une homologie avec le répresseur λ d'*E.coli* ; région conférant la spécificité de promoteur) et celles impliquées dans le site catalytique (motif A ; motif B ; motif C).

Pour la définition des motifs A, B et C, voir par exemple R. Sousa, TIBS 21, 186-190 (1996).

Ces cassettes, dérivées du gène *1* de la T7 ARNpADNd, ont été obtenues à l'aide de techniques de biologie moléculaire conventionnelles (F. M. Ausubel, R. Brent, R. E. Kingston, D. D. Moore, J. G. Seidman, et al, Current *Protocols in Molecular Biology* (Current Protocols, 1993)), notamment la PCR, qui a permis d'introduire des sites de restriction par mutagénèse dirigée silencieuse, et le sous-clonage dans un vecteur de clonage.

Le gène modulaire ainsi obtenu est caractérisé par la présence de sites de restrictions bordant les cassettes, ces sites de restriction étant le site Nco I (-2,+4), Bcl I (218,223), Hind III (539,544), SacI (776,781), PstI (1587, 1592), BglII (1811, 1816), NdeI (1865, 1870), XhoI (1951,1956), ClaI (2305, 2310), SalI (2497, 2502), XbaI (2660, 2665) ; la position 1, en acides nucléiques, correspond à l'adénine du codon ATG initiateur et la position 2652 à la troisième base du codon terminateur TAA. Le site NdeI (2504, 2509) a été détruit. Toutes les mutations induisant ces sites de restriction sont silencieuses, excepté la mutation générant le site NcoI qui induit le remplacement de l'asparagine en position +2 par une glycine. La position 1, en acides aminés, correspond à la première méthionine, la position 883 correspond à l'alanine carboxy-terminale.

Le gène modulaire, cloné dans un vecteur de clonage pGNEX dérivé du pGEM-1 dans lequel le polylinker a été remplacé par un adaptateur contenant les sites de restriction Nco I, EcoRI, XbaI, constitue le support de base des mutagénèses ultérieures. Ceci est rendu possible par le fait que chaque cassette contenant une région d'intérêt est bordée par des sites de restriction uniques dans le vecteur de clonage.

L'introduction de mutations non silencieuses, à l'aide de techniques de PCR, dans une ou plusieurs cassettes du gène modulaire précédemment défini, a conduit à des gènes codant pour des polymérases présentant une séquence en acides aminés qui diffère d'au moins un acide aminé par rapport à la T7 exprimée à partir du gène modulaire. On a préparé en particulier des gènes mutants qui codent pour au moins un acide aminé modifié dans le motif B de l'enzyme sauvage, par exemple avec une alanine (A) à la place d'une arginine (R) en position 627 et/ou une alanine (A) à la place d'une sérine (S) en position 628 et/ou une alanine (A) à la place d'une lysine (K) en position 631 et/ou une alanine (A) à la place d'une arginine (R) en position 632 et/ou une alanine (A) à la place d'une tyrosine (Y) en position 639.

On a également obtenu des gènes mutants qui codent pour une polymérase dont la région 625VTRSVTKRSVMTLAYGSKEFGFRQQVLD652 comprenant le motif B a été remplacée en tout ou partie par la région homologue B' présente chez certaines polymérases ARN dépendantes, notamment celles des polymérases du virus de l'hépatite C (NCGYRRCRASGVLTTSCGNTLTCYI), et de l'intégrase 32 de levure (HNTTLGIPQGSVVSPILCNIFLDKL).

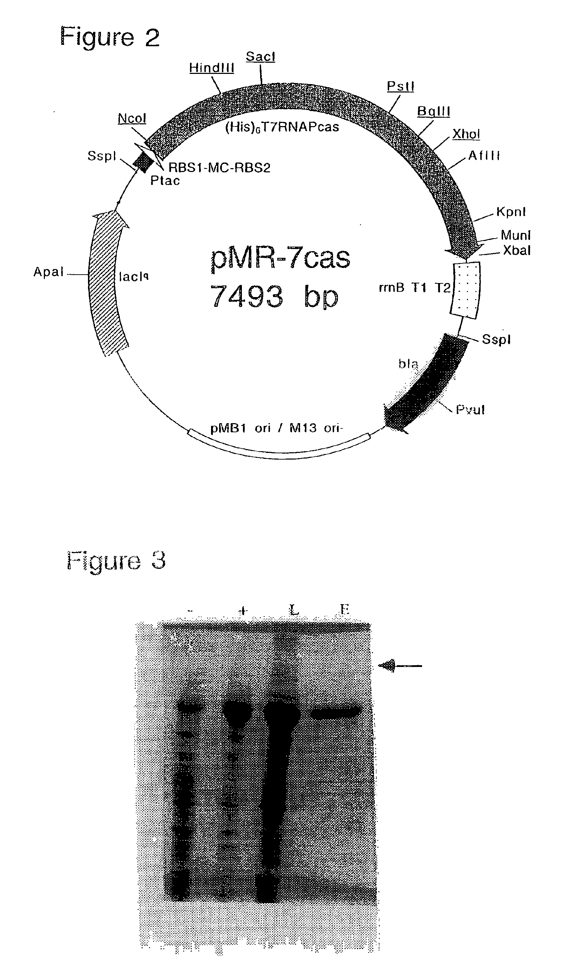

Les gènes précédemment décrits ont été clonés dans un vecteur pMR résultant de la ligation du fragment SspI du pMal-c (Biolabs) contenant notamment le répresseur lacI^{q}, et du fragment SspI du pMH (V. Cheynet, B. Verrier, F. Mallet, *Protein expression and purification* **4**, 367 (1993)) contenant un minicistron permettant d'atteindre un haut niveau d'expression, ainsi qu'une séquence codant pour une queue poly-histidine fusionnée à l'extrémité terminale du gène cloné (exemple 1, figure 2). L'expression des protéines recombinantes T7 ARNp dans la souche bactérienne BL21 représente jusqu'à 30 % des protéines totales de la bactérie. Les protéines solubilisées dans un tampon désoxycholate contenant une forte concentration saline sont déposées sur une colonne TALON (Clontech) permettant la purification spécifique par chélation avec l'ion de protéines présentant une queue polyhistidine. 130 à 2200 µg de polymérases sont ainsi obtenus pour 20 ml de culture, avec une pureté supérieure à 95 % comme indiqué (i) par une coloration au bleu de Coomassie (exemple 1, figure 3), (ii) par une analyse en Western-blot avec un anticorps polyclonal de cobaye anti-T7 RNAP (bioMérieux) et un anticorps monoclonal de souris (Quiagen) anti-MRGSHHHHHH, (iii) par l'absence d'activité endonucléase, exonucléase simple brin et double brin, ribonucléase, comme déterminé essentiellement selon la méthode de He et al (B. A. He, M. Rong, D. L. Lyakhov, H. Gartenstein, G. Diaz, et al, *Protein Expr Purif* **9**, 142 (1997)). Ce résultat reflète les performances du couple hôte-vecteur pMR-BL21 et du procédé de purification vis-à-vis de la queue MRGSHHHHHSVLE.

L'invention a également pour objet l'utilisation d'une ARN polymérase telle que définie précédemment, dans un procédé de transcription d'un segment polynucléotidique d'intérêt ayant une séquence quelconque, ledit segment, de type ARN, étant contenu dans une matrice polynucléotidique, en vue de synthétiser, en présence de ladite matrice, un produit de transcription contenant une séquence d'ARN complémentaire de la séquence dudit segment polynucléotidique d'intérêt.

Selon un mode d'exécution particulier, ladite utilisation est caractérisée par le fait que ladite matrice polynucléotidique comprend, en amont dudit segment polynucléotidique d'intérêt, un promoteur reconnu par ladite ARN polymérase, et que ledit produit de transcription est un ARN complémentaire d'une séquence de la matrice commençant en un site d'initiation de la transcription pour ledit promoteur.

Les ARN polymérases de l'invention peuvent être utilisées notamment en vue de réaliser (i) une amplification de cible ARN de façon isotherme, (ii) un séquençage direct d'ARN et (iii) la synthèse d'ARN d'intérêt particulier (par exemple des sondes, des ribozymes, etc.). En outre, des ARN polymérases de l'invention sont capables d'incorporer des bases modifiées dans le brin néo-synthéthisé, ce qui facilite notamment la quantification ou l'utilisation dudit brin.

L'invention concerne notamment l'utilisation de ces enzymes recombinantes ainsi exprimées et purifiées dans une méthode de synthèse d'ARN à partir d'une matrice ARN, sous la dépendance d'un promoteur.

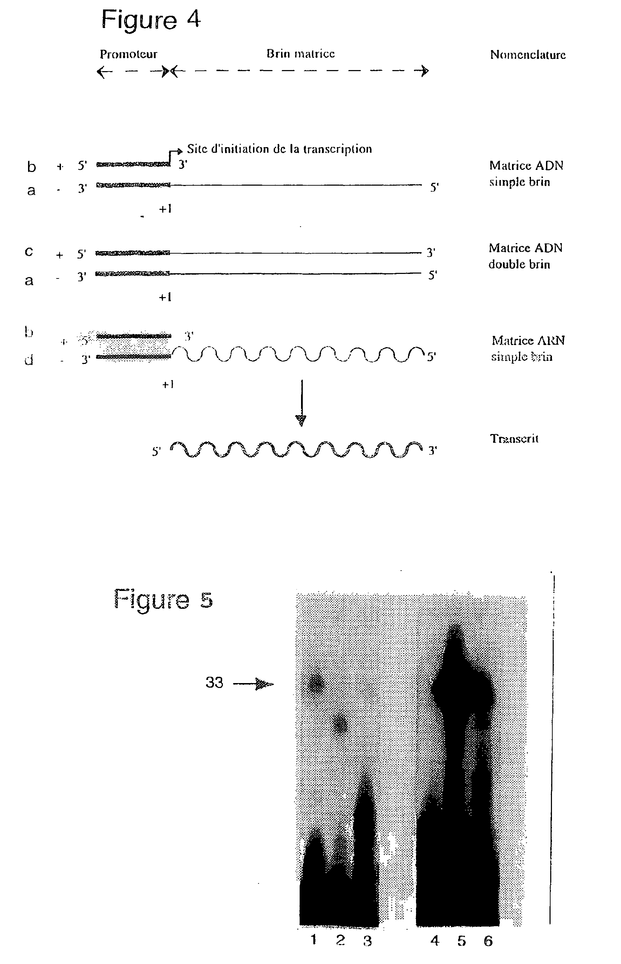

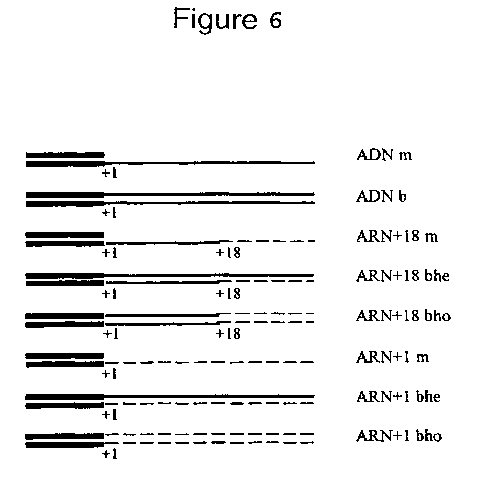

Les enzymes ainsi purifiées ont été évaluées, dans un contexte promoteur-dépendant, sur différentes matrices (exemple 2, figure 4 d'une part, et exemple 3, figure 6, d'autre part) en particulier une matrice comportant un ARN simple brin. Il a été montré qu'une polymérase mutée obtenue selon l'invention était capable de générer un transcrit spécifique de la bonne taille notamment sur matrice ARN simple brin. Dans l'exemple 2, il est indiqué que l'enzyme sauvage identiquement produite ne semble pas pouvoir réaliser ce phénomène. Toutefois, l'exemple 3 montre que l'enzyme sauvage possède en fait la propriété de transcrire une matrice d'ARN. Ces résultats apparemment divergents s'expliquent par le fait que les conditions expérimentales, dans ces deux exemples sont différentes. En effet, dans l'exemple 2, la présence du transcrit est mise en évidence par la technique d'incorporation d'un UTP, marqué au phosphore radioactif, tandis que dans l'exemple 3 la technique utilisée est le Northern Blot pour les matrices du groupe 2, et dans ce dernier cas, la détection du transcrit par la technique Northern Blot est 40 fois plus sensible que la détection par incorporation de phosphore radioactif. L'exemple 2 ci-après montre donc qu'une polymérase mutée obtenue selon l'invention est capable de générer un transcrit spécifique de la bonne taille sur matrice ARN simple brin, et l'exemple 3 montre qu'en fait la polymérase sauvage correspondante est capable de générer un transcrit spécifique de la bonne taille sur des matrices ARN indépendamment de leur séquence.

De plus, une telle polymérase mutée est incapable, contrairement à la polymérase sauvage, de générer un transcrit de bonne taille sur matrice ADN simple brin ou double brin. Si on remplace l'ion Mg²⁺ présent dans le milieu réactionnel par l'ion Mn²⁺, l'enzyme mutée, sur matrice ARN simple brin, ne génère pas dans ces conditions de transcrit spécifique de la bonne taille, mais est cependant capable de générer de grandes quantités de produits abortifs. Une telle polymérase mutée est en outre capable de déplacer un hybride ARN/ARN.

Dans les dessins annexés .
- la figure 1 illustre la stratégie d'amplification, décrite en détail à l'exemple 1 ci-après, pour la construction d'un gène modulaire de la T7 ARN polymérase,
- la figure 2 illustre la structure du vecteur d'expression pMR contenant ce gène modulaire,
- la figure 3 représente des profils d'électrophorèse des protéines exprimées à l'aide de ce vecteur, comme décrit en détail à l'exemple 1 ci-après,
- la figure 4 représente schématiquement les systèmes matriciels utilisés dans les essais de transcription de l'exemple 2 ci-après,
- la figure 5 représente les profils d'electrophorèse des produits de transcription obtenus à l'exemple 2 ci-après, et
- la figure 6 représente schématiquement les systèmes matriciels utilisés dans les essais de transcription de l'exemple 3 ci-après.

Les exemples suivants illustrent l'invention.

### EXEMPLES

### Exemple 1 : Construction du gène de la T7 ARN polymérase sous forme modulaire ; expression et purification de la T7 ARN polymérase.

Le gène de la T7 ARN polymérase a été construit sous forme modulaire, en tenant compte des régions d'homologie avec les autres polymérases ainsi que des fonctions associées à certains domaines de la T7 ARN polymérase. Il a été divisé en 10 régions ou cassettes (Figure 1-A), chaque région étant délimitée par des sites de restriction uniques dans le vecteur de clonage. Six cassettes sont caractéristiques en ce qu'elles contiennent respectivement un domaine ayant une similarité avec une partie du facteur sigma d'*E.coli,* un domaine ayant une homologie avec le répresseur lambda d'*E.coli,* un domaine impliqué dans la spécificité de promoteur des polymérases phagiques, les motifs A et C conservés chez les polymérases matrices dépendantes et le motif B conservé chez les polymérases ADN-dépendantes. Chaque région est amplifiée par PCR (F. M. Ausubel, R. Brent, R. E. Kingston, D. D. Moore, J. G. Seidman, et al, *Current Protocols in Molecular Biology* (Current Protocols, 1993)) (Figure 1a-B1) à partir du gène sauvage (gène *1*) en utilisant des amorces définies comme suit :
(i) ces amorces contiennent des mutations silencieuses permettant l'introduction de sites de restriction délimitant les cassettes ;
(ii) l'amorce située en position 3' d'une région et l'amorce située en position 5' de la région contiguë sont partiellement chevauchantes. Les produits d'amplification contigus sont mélangés et réamplifiés en utilisant les amorces externes (Figure la-B1). Les 5 fragments générés sont clonés dans le vecteur PCRII (Invitrogen) (Figure 1-B2). A partir des 5 fragments clonés, le gène de la T7 ARN polymérase est reconstruit de l'extrémité 5' vers l'extrémité 3', par des sous-clonages successifs dans le vecteur de clonage pGNEX (Figure 1-B3). Ce vecteur est dérivé du plasmide pGEM-1 (Promega) dans lequel le polylinker est remplacé par un adaptateur contenant les sites de restriction NcoI-EcoRI-XbaI. Le gène modulaire de la T7 ARN polymérase (T7 ARN pol) ainsi obtenu contient 17 sites uniques de clonage dans le pGNEX dont 11 sites nouvellement introduits (Figure 1-C). Ce gène est ensuite sous-cloné dans le vecteur d'expression pMR (Figure 2). Ce vecteur, dérivé du vecteur pMH (V. Cheynet, B. Verrier, F. Mallet, *Protein expression and purification* **4**, 367 (1993)), contient le promoteur fort *tac* régulé par IPTG (isopropyl-bêta-D-thiogalactopyranoside), un court minicistron entouré de 2 sites de liaison des ribosomes constituant un contexte efficace pour l'initiation de la traduction et une queue polyhistidine fusionnée à l'extrémité N-terminale de la protéine exprimée permettant sa purification par chromatographie d'affinité pour un ion métallique. Le gène codant pour le répresseur lacI^{q} permettant un meilleur contrôle de l'expression est également présent dans pMR. Ce vecteur d'expression est transformé selon les méthodes classiques (F. M. Ausubel, R. Brent, R. E. Kingston, D. D. Moore, J. G. Seidman, et al, Current *Protocols in Molecular Biology* (Current Protocols, 1993)) dans la souche bactérienne E.coli BL21. Une préculture est utilisée pour inoculer 30 ml de milieu de culture. L'expression des protéines est induite par IPTG pendant 4 h à 37°C dès que la densité optique à 600 nm de la culture atteint 0,6. La T7 ARN pol ainsi exprimée représente 30 % des protéines bactériennes totales (Figure 3). Elle est ensuite extraite des bactéries par lyse (sonication) en présence d'un détergent. A partir de la fraction d'extraction soluble, la T7 ARN pol est purifiée par chromatographie d'affinité pour l'ion Co²⁺ et éluée par un gradient d'imidazole. Elle est analysée sur un gel SDS-PAGE et visualisée par coloration au bleu de Coomassie (Figure 3). L'absence de produits de dégradation est vérifiée par Western Blot avec un anticorps polyclonal anti T7 ARN pol produit chez le cobaye et un anticorps monoclonal anti MRGSH₆ commercial (Qiagen). L'absence d'activités parasites endonucléase, exonucléases et RNase est vérifiée selon le protocole décrit par He et al , *Protein Expr Purif* **9**, 142 (1997)). A partir de 20 ml de culture, 700 à 800 µg d'enzymes, de pureté supérieure à 95 %, sont obtenus de manière reproductible. Le rendement de purification est de 75 %.

Les mutations ponctuelles sont créées par PCR séquentielle (F. M. Ausubel, R. Brent, R. E. Kingston, D. D. Moore, J. G. Seidman, et al, *Current Protocols in Molecular Biology* (Current Protocols, 1993)) (ou double PCR), de la même façon qu'avaient été introduits les sites de restriction: les amorces internes contiennent la mutation à introduire ; les amorces externes encadrent la région à muter et contiennent les sites de restriction délimitant cette région dans le gène modulaire. Cette cassette ainsi mutée est ensuite clonée dans le gène modulaire. Des oligonucléotides synthétiques peuvent également remplacer une cassette complète.

### Légende des figures mentionnées à l'exemple 1 :

**Figure 1** : illustre la stratégie d'amplification pour la construction du gène modulaire de la T7 ARN polymérase. **(A)** : Division du gène de la T7 ARN polymérase (flèches verticales) par rapport aux régions d'intérêt. Les régions notées de 1 à 6 sont : 1, homologie avec une partie du facteur sigma d'*E.coli ;* 2, homologie avec le répresseur lambda d'*E.coli* ; 3, motif A ; 4, motif B ; 5, spécificité de promoteur des polymérases phagiques ; 6, motif C. **(B) : (1)** Position des amorces contenant les mutations silencieuses pour la création des sites de restriction ( 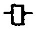 ) ; Nc, NcoI ; Bc, BclI ; H, HindIII ; Sc, SacI , P, PstI ; Bg, BglII ; Nd, NdeI ; Xo, XhoI ; C, ClaI ; Sl, SalI ; Xb, XbaI. L'extension par PCR chevauchante conduit à 5 fragments contenant 3 sites de restriction (F1 à F5). Sub-cloning signifie : sous-clonage. **(2)** Chaque fragment ainsi généré (F1 à F5) est cloné dans le vecteur pCRII. E, EcoRI. **(3)** Reconstruction du gène modulaire de l'extrémité 5' vers l'extrémité 3', en utilisant à chaque étape le site de restriction commun à 2 fragments et le site de restriction EcoRI du vecteur pCRII et du vecteur de clonage pGNEX ; le site de restriction XbaI commun au dernier fragment et au vecteur pGNEX est utilisé pour le dernier sous-clonage. (C) Structure du gène modulaire de la T7 ARN polymérase et position des sites de restriction uniques dans le vecteur pGNEX déjà existants (en bas) et créés par mutagénèse (en haut).

**Figure 2 :** illustre la structure du vecteur d'expression pMR contenant le gène modulaire de la T7 ARN polymérase. Ptac : promoteur tac (boîte noire) ; RBS1-MC-RBS2, minicistron entouré de 2 sites de liaison des ribosomes (flèche blanche) ; (His)6T7RNAPcas, gène modulaire de la T7 ARN polymérase (flèche grise) ; rrnB T1 T2, terminateurs de transcription forts (boîte avec pointillés) ; bla, gène de résistance à l'ampicilline (flèche noire) ; pMB1 ori/M132 ori-, origines de réplication (fine boîte blanche) ; lacI^{q}, gène codant pour le répresseur lacI^{q} (flèche rayée) ; certains sites de restriction sont indiqués, dont les nouveaux sites introduits par mutagénèse (soulignés).

**Figure 3** : montre les profils électrophorétiques obtenus permettant l'analyse de l'expression et de la purification de la T7 ARN polymérase. Les lysats bactériens sont préparés à partir de 1ml prélevés dans 30 ml de culture pour contrôler l'expression de la T7 ARN polymérase en présence (+) ou absence (-) d'IPTG. La T7 ARN polymérases est extraite à partir de 20 ml de la même culture. La fraction soluble est chargée sur la colonne de purification (piste L) et la T7 ARN polymérase est éluée (piste E) par un gradient d'imidazole. M, marqueur de poids moléculaire. Les protéines sont visualisées sur un gel 10 % SDS-PAGE par coloration au bleu de Coomassie. La flèche indique la position de la T7 ARN polymérase.

### Exemple 2 : Transcription de matrices ARN et ADN par la T7 ARN polymérase mutée R627A sous la dépendance d'un promoteur

Les systèmes matriciels utilisés dans cet exemple sont présentés schématiquement figure 4. Le système de transcription ADN simple brin comprend un brin matrice (a) de 50 bases de séquence 3'ATTATGCTGAGTGATATCCCAACCGGCGTCACAAGTGAGTACCAATACCGS' et hybridé des positions -17 à +1 au brin promoteur non-matrice (b) de séquence 5'TAATACGACTCACTATAG 3'(bloqué à son extrémité 3'). Le système de transcription ADN double brin comprend le brin matrice (a) hybridé au brin non matrice (c) de séquence 5'TAATACGACTCACTATAGGGTTGGCCGCAGTGTTCACTCATGGTTATGGC3'.

Le système de transcription ARN simple brin est formé d'un brin hybride ADN des positions -17 à -1 et ARN des positions +1 à +33 de séquence 3'ATTATGCTGAGTGATATCCCAACCGGCGUCACAAGUGAGUACCAAUACCG5', hybridé au brin promoteur non-matrice (b). Les transcrits complets attendus sur les trois systèmes sont de 33 bases.

Les réactions sont faites dans 20 µl d'un tampon dérivé de celui décrit par J. F. Milligan, D. R. Groebe, G. W. Witherell, O. C. Uhlenbeck, Nucleic Acids Res. 25, 8783 (1987), à savoir Tris-HCl 40 mM, pH 8,1, spermidine 1mM, PEG 8 % (g/V), triton 0,01 % (V/V), BSA 5 µg/100 µl, 1 µl (40 u) de RNAguard porcine (Pharmacia Biotech), UTP 12,5 µM, a 32P UTP 0,5 µCi (Amersham, 10 mCi/ml 400Ci/mmol) 0,4 mM des trois ribonucléosides triphosphate A, G, C, Mg(OAc)₂ 6 mM. La concentration en matrice est fixée à 10¹¹ copies de chaque brin dans 20 µl de réaction. La T7 ARN polymérase sauvage est utilisée à 0,5 µM (100 ng / 20 µl), la T7 ARN polymérase mutée R627A à 3,65 µM (730 ng / 20 µl). Avant d'ajouter les enzymes, les réactions sont dénaturées 5 minutes à 65°C dans un bloc chauffant puis ramenées progressivement à 37°C. Les réactions sont initiées par l'ajout des polymérases, incubées 1 heure à 37°C puis stoppées par ajout d'un volume égal de bleu formamide 2x (formamide 90 %, EDTA 25 mM, xylène cyanol 0,02 %, bleu de bromophénol 0,02 %) et dénaturées 5 minutes à 95°C. 20 µl de chaque réaction sont déposés sur gel dénaturant (20 % acrylamide, urée 7 M, TBE 1X), puis après migration le gel est autoradiographié à -70°C sur film Biomax MR (Kodak). Les résultats (profils d'électrophorèse) sont présentés figure 5, et en particulier les résultats de transcription obtenus avec la T7 ARN polymérase mutée R627A (puits 1-3) et la T7 ARN polymérase sauvage (puits 4-6), sur les matrices ARN simple brin (puits 1 et 4), ADN double brin (puits 2 et 5), et ADN simple brin (puits 3 et 6). La transcription sur ARN simple brin, détectée par la détection d'un transcrit complet de 33 bases, est possible en utilisant la T7 ARN polymérase mutée R627A (puits 1) et non l'enzyme sauvage (puits 4) qui produit par contre de nombreux transcrits abortifs ; voir cependant les résultats différents obtenus à l'exemple 3 ci-après. La T7 ARN polymérase mutée R627A présente une activité de transcription résiduelle sur ADN double brin (puits 2), caractérisée par la présence d'un transcrit majoritaire de taille inférieure au transcrit attendu, et la présence en faible quantité de produits abortifs. Sur ADN simple brin (puits 3), ce transcrit de taille anormale disparaît, alors que la quantité de produits abortifs augmente. Au cont-raire, l'enzyme sauvage permet l'obtention de transcrits spécifiques en présence de matrices ADN (puits 5 et 6), cette enzyme présentant par ailleurs une meilleure activité de transcription sur la matrice ADN double brin (puits 5) que sur la matrice ADN simple brin (puits 6) ; pour ces deux matrices, l'enzyme sauvage induit la synthèse de nombreux transcrits abortifs. Ces résultats montrent que le remplacement de l'arginine 627 par une alanine confère à l'enzyme mutante la possibilité de synthétiser de l'ARN à partir d'une matrice ARN et induit la perte de capacité à synthétiser de l'ARN à partir d'une matrice ADN.

### Exemple 3 :

On purifie la T7 ARN polymérase obtenue comme à l'exemple 1 par chromatographie d'affinité selon la méthode décrite par Arnaud N. et al., *Gene*, **199**, 149-156 (1997).

La T7 ARN polymérase purifiée a une activité spécifique d'environ 200 U/µg.

Les séquences des matrices utilisées pour la transcription sont décrites dans le tableau 1 ci-après.

Dans les essais décrits ci-dessous, les matrices de séquence 1, 2 et 3 du tableau 1 sont appelées matrices du groupe 1, du groupe 2 et du groupe 3, respectivement.

La séquence des sondes utilisées est représentée dans le tableau 1 (séquences n° 4 et 5).

La séquence n° 4 reconnaît l'extrémité 3' des produits de transcription obtenus avec les matrices des groupes 1 et 3, et la séquence n° 5 reconnaît l'extrémité 3' des produits de transcription de la matrice du groupe 2.

Les sondes sont marquées avec un γ³²P ATP par la T4 polynucléotide kinase ou avec un α³²P ddATP par une désoxynucléotide terminal transferase.

### Essais de transcription

Les réactions sont effectuées dans un volume de 20 µL contenant 40 m/M Tris- HC1, ImM spermidine 50 µg/ml d'albumine sérique bovine, 0,01 % (v/v) Triton X100, 80 mg/ml PEG 8000, 1 µl RNAguard (Pharmacia), 6 mM acétate de magnésium, 10¹¹ copies des brins matrice et non matrice, les nucléosides triphosphates nécessaires à la transcription et les nucléosides triphosphates marqués comme indiqué. Lorsque le nucléoside triphosphate marqué est le α³²P ATP, on utilise des concentrations de 0,4 mM UTP, CTP et GTP, 12,5 µM ATP et 0,5 µCi α³²P ATP (New England Nuclear-Dupont, 800 Ci/mmol). Lorsque le nucléoside triphosphate marqué est le α³²P UTP, on utilise des concentrations de 0,4 mM ATP, CTP et GTP, 12,5 µM d'ATP et 0,5 µCi α³²P UTP (Amersham, 400 Ci/mmol). Lorsque le nucléoside triphosphate marqué est le γ³²P GTP, on utilise des concentrations de 0,4 mM ATP, UTP, CTP et GTP, et 20 µCi γ³²P GTP (Amersham, 5000 Ci/mmol) .

Les échantillons sont chauffés pendant 5 minutes à 70°C puis refroidis lentement jusqu'à 37°C pour permettre l'hybridation des brins matrice et non matrice. On ajoute ensuite 260 ng de T7 ARN polymérase et on laisse incuber pendant 1 heure à 37°C. On arrête les réactions en ajoutant un égal volume de tampon 2X (90 % formamide, 25 mM EDTA, 0,02 % xylène cyanol, 0,02 % bleu de bromophénol). Les produits de transcription sont analysés par électrophorèse, après chauffage pendant 5 minutes à 95°C, sur un gel dénaturant à 20 % de polyacrylamide, et examinés par autoradiographie.

Pour l'analyse Northern Blot, on effectue les réactions dans les mêmes conditions, mais sans les nucléotides marqués. Les échantillons, après migration sur un gel dénaturant à 20 % de polyacrylamide, sont transférés sur membrane de nylon (Appligene) et les produits de transcription sont détectés par la sonde marquée appropriée et sont examinés par autoradiographie.

### Définition de matrices synthétiques courtes

Trois types de matrices synthétiques courtes, contenant un promoteur ADN bicaténaire , ont été définis afin de vérifier si la T7 ARN polymérase est capable d'utiliser une matrice d'ARN dans une réaction de transcription.

Le premier type de matrices (ARN+18) a été défini afin d'étudier là capacité de la T7 ARN polymérase à transcrire une matrice ARN durant la phase dite processive. Ce premier type de matrice comprend un promoteur bicaténaire suivi en aval par une séquence chimère ADN-ARN dont la transition est située 18 bases en aval du site d'initiation de la transcription.

Le second type de matrices (ARN+1) a été défini afin d'étudier la capacité de la T7 ARN polymérase à transcrire une matrice ARN durant la phase de début de transcription. Ce second type de matrices comprend un promoteur bicaténaire suivi d'une séquence d'ARN.

Le troisième type de matrices (ADN), qui sert de comparaison, comprend un promoteur bicaténaire suivi en aval d'une séquence d'ADN.

Afin d'étudier l'influence du brin non matrice, les matrices ADN, ARN+18 et ARN+1 peuvent être soit monocaténaires (m), soit des hétéroduplex bicaténaires (bhe), soit encore des homoduplex bicaténaires (bho). La matrice homoduplex bicaténaire ARN+18 forme un duplex ARN-ARN à partir de la position +18. De même la matrice homoduplex bicaténaire ARN+1 forme un duplex ARN-ARN à partir du site de début de la transcription.

Il convient de noter que dans le tableau 1, on a représenté les différents nucléotides avec les lettres A, T, C, G. Lorsque la matrice, ou une partie de la matrice, est de l'ADN, ces lettres représentent des désoxyribonucléotides. Lorsque la matrice, ou une partie de la matrice, est en ARN, ces lettres représentent des ribonucléotides et il faut comprendre que le symbole T est alors utilisé en remplacement du symbole U.

Les différents systèmes de transcription qui viennent d'être évoqués sont représentés schématiquement dans la figure 6 annexée dans laquelle, pour chaque système bicaténaire, le brin supérieur est le brin non matrice et le brin inférieur est le brin matrice. La région promotrice double brin ADN est représentée par un trait épais. Les brins ADN sont représentés par un trait fin, les brins ARN sont représentés par un trait interrompu. L'indication +1 correspond au site de début de transcription. L'indication +18 correspond à la dix-huitième base en aval du site de début de la transcription. Dans la désignation de ces différents systèmes de transcription, la lettre m signifie monocaténaire ; la lettre b signifie bicaténaire ; bhe signifie hétéroduplex bicaténaire ; et bho signifie homoduplex bicaténaire.

Les systèmes de transcription de la figure 6 ont été utilisés avec chacune des matrices, ou, selon les cas, avec certaines des matrices du tableau 1. Les matrices du groupe 1, utilisant la séquence n° 1, correspondent à une séquence promotrice d'ADN double brin suivie d'une région d'initiation +1 à +6 consensus (Dunn et Studier, J. Mol. Biol., 166, 477-535, 1983) elle-même suivie d'une séquence de vingt six bases en aval.

Les matrices du groupe 2 correspondent à une séquence promotrice ADN double brin suivie d'une séquence non favorable, puisqu'elle peut permettre la terminaison précoce de la transcription (Martin et al., *Biochemistry,* **27**, 3966-3974, 1988).

Les matrices du groupe 3 correspondent à la même séquence d'initiation non favorable que le groupe 2, suivie de la même séquence aval que le groupe 1.

### Résultats

Les résultats sont résumés dans le tableau 2 ci-joint.

On constate que la transition ADN-ARN sur la matrice ARN+18 est efficacement passée par le complexe d'élongation, comme le montre l'absence d'accroissement de terminaisons précoces autour de la position +18, par rapport au témoin ADN.

La T7 ARN polymérase est capable d'initiation de la transcription sur différentes matrices d'ARN et est capable de transcrire entièrement ces matrices. La détection de transcrits ayant la taille attendue, pour tous les groupes de matrices, montre que l'utilisation de la matrice ARN n'est pas dépendante de la séquence, bien que l'efficacité globale dépende de la composition en bases, comme cela est observé aussi sur les matrices ADN témoins.

Les résultats différents mentionnés à l'exemple 2 ci-dessus proviennent du fait que la détection par la technique Northern Blot est dans le cas présent 40 fois plus sensible que la technique de détection utilisée à l'exemple 2, comme cela a été signalé ci-dessus dans la description.

La T7 ARN polymérase est capable de transcrire un duplex ARN-ARN. Aucun accroissement du nombre de produits de transcription abortifs n'est observé avec la matrice homoduplex ARN+1.

La transcription des matrices bicaténaires homo- ou hétéro-duplex est globalement similaire. Dans le cas des matrices ARN+18 du groupe 2, on obtient davantage de transcrits de la taille attendue avec le système homoduplex qu'avec le système hétéroduplex.

La présence du brin non matrice influence l'efficacité de la transcription. En effet, le rendement de transcription est accru sur les matrices monocaténaires, comme le montrent, d'une part, l'augmentation du nombre de transcrits de bonne taille et, d'autre part, le fait qu'un événement d'initiation soit plus fréquemment associé à la synthèse d'un transcrit complet. Cependant, avec les matrices du groupe 2, le système monocaténaire ne donne pas de meilleurs résultats.

Ainsi, la T7 ARN polymérase possède une activité transcriptionnelle sur matrice ARN, en présence d'un promoteur bicaténaire ADN. Le rendement de transcription observé n'est que 10 à 100 fois plus faible que sur matrice ADN.

**TABLEAU 1**

| |
|---|
| SEQUENCE N° 1 : 3'ATTATGCTGAGTGATATCCCTCTAGATCGTCGTATTGCGAGGTTCATGT    5' +1                    +18 |
| SEQUENCE N° 2 : 3'ATTATGCTGAGTGATATCCCAACCGGCGTCACAAGTGAGTACCAATACCG 5' +1 +18 |
| SEQUENCE N° 3 : 3'ATTATGCTGAGTGATATCCCAACAGATCGTCGTATTGCGAGGTTCATGT 5' +1 +18 |
| SEQUENCE N° 4 : |
| 3' ATTGCGAGGTTCATGT 5' |
| SEQUENCE N° 5: |
| 3' GTGAGTACCAATACCG 5' |

**TABLEAU 2**

| | Groupe (1) | | | Groupe (2) | | | Groupe (3) | | |
|---|---|---|---|---|---|---|---|---|---|
| Matrice | TS^{a} | EI^{b} | Eff^{c} | TS^{a} | EI^{b} | Eff^{c} | TS^{a} | EI^{b} | Eff^{c} |
| ADN m | 240 | 242 | 1/10 | 192 | 593 | 1/31 | 149 | 633 | 1/43 |
| ADN bho | 80 | 152 | 1/18 | 102 | 191 | 1/19 | 67 | 893 | 1/133 |
| ARN+18 m | 800 | 650 | 1/8 | 326 | 1488 | 1/45 | 253 | 1246 | 1/49 |
| ARN+18 | 208 | 309 | 1/14 | 99 | 647 | 1/65 | 61 | 642 | 1/106 |
| bhe | | | | | | | | | |
| ARN+18 | 176 | 272 | 1/16 | 358 | 1093 | 1/31 | nr^{e} | nr | nr |
| bho | | | | | | | | | |
| ARN+1 m | 13 | 141 | 1/108 | NA^{f} | NA | NA | NA^{d} | NA | NA |
| ARN+1 bhe | 3.2 | 61 | 1/227 | NA^{d} | NA | NA | NA^{d} | NA | NA |
| ARN+1 bho | 6.4 | 110 | 1/157 | NA^{d} | NA | NA | nr | nr | nr |

| | | | | | | | | | |
|---|---|---|---|---|---|---|---|---|---|
| Comparaison de la transcription par la polymérase T7 sur différentes matrices, par incorporation de γ³²P-GTP ^{a}Ts : transcrit spécifique en picomoles pour 10 picomoles de matrice ^{b}EI : nombre d'événements d'initiation pour une copie de matrice ^{c}Eff : l'efficacité correspond au nombre d'événements d'initiation conduisant à la synthèse d'un transcrit complet NA: non accessible (non quantifiable) d : non accessible; cependant le transcrit complet est détecté en Northern blot nr: non réalisé | | | | | | | | | |

## Revendications

1. Procédé d'amplification d'une séquence cible quelconque d'ARN, par transcription sous la dépendance d'un promoteur, dans un échantillon d'ARN comprenant ladite séquence cible, dans lequel on met en contact ledit échantillon
- avec un réactif capable de s'hybrider avec ledit ARN comprenant ladite séquence cible,
- et avec un système enzymatique comprenant une activité d'ARN polymérase ARN-dépendante,
dans des conditions permettant l'hybridation dudit réactif avec ledit ARN comprenant ladite séquence cible et dans des conditions permettant le fonctionnement de ladite activité d'ARN polymérase ARN-dépendante ; dans lequel ledit réactif contient
(i) un premier brin nucléotidique comprenant : a) un premier segment nucléotidique capable de jouer le rôle de brin sens d'un promoteur pour ladite activité d'ARN polymérase et b), en aval dudit premier segment, un second segment nucléotidique comprenant une séquence capable d'hybridation avec une région dudit ARN, et
(ii) à l'état hybridé sur le premier brin, un second brin nucléotidique comprenant un troisième segment nucléotidique capable d'hybridation avec ledit premier segment de façon à former avec lui un promoteur double brin fonctionnel ;
et dans lequel ladite activité d'ARN polymérase est capable de transcrire une matrice d'ARN, en présence dudit réactif hybridé sur ladite matrice, en l'absence de facteur protéique associé et en l'absence d'une activité de ligase.

2. Procédé selon la revendication 1, dans lequel ledit troisième segment est flanqué, à son extrémité amont, par un quatrième segment nucléotidique qui est plus court que ledit second segment du premier brin.

3. Procédé selon la revendication 2, dans lequel ledit quatrième segment est capable d'hybridation avec une partie en regard dudit second segment.

4. Procédé selon l'une quelconque des revendications 2 et 3, dans lequel ledit quatrième segment dudit second brin est choisi parmi ceux dont la séquence facilite l'initiation de la transcription pour ladite ARN polymérase.

5. Procédé selon l'une quelconque des revendications 2 à 4, dans lequel ledit second segment dudit premier brin contient un nombre de nucléotides au moins égal à la somme du nombre de nucléotides dudit quatrième segment, s'il est présent, et du nombre de nucléotides de ladite séquence du second segment qui est capable d'hybridation avec ladite région dudit ARN.

6. Procédé selon l'une quelconque des revendications précédentes, dans lequel lesdits premier et troisième segments sont constitués d'ADN.

7. Procédé selon l'une quelconque des revendications précédentes, dans lequel ledit quatrième segment est constitué d'ADN.

8. Procédé selon l'une quelconque des revendications précédentes, dans lequel ladite ARN polymérase est une ARN polymérase de type sauvage de virus ou de phage.

9. Procédé selon la revendication 8, dans lequel ladite ARN polymérase est choisie dans la famille des ARN polymérases incluant la T7 ARN polymérase, la T3 ARN polymérase et la SP6 ARN polymérase.

10. Procédé selon la revendication 8, dans lequel ladite ARN polymérase dérive par mutation d'une ARN polymérase choisie dans la famille des ARN polymérases incluant la T7-, la T3 et la SP6 ARN polymérases.

11. Procédé selon la revendication 10, dans lequel ladite ARN polymérase contient au moins une mutation dans la région correspondant à la séquence d'acides aminés 625 à 652 de la T7 ARN polymérase.

12. Procédé selon la revendication 11, dans lequel ladite ARN polymérase est capable de transcrire une séquence cible poly- nucléotidique avec un meilleur rendement lorsque ladite séquence cible est constituée d'ARN que lorsqu'elle est constituée d'ADN.

13. Procédé selon l'une quelconque des revendications précédentes, dans lequel ledit système enzymatique contient uniquement une activité d'ARN polymérase.

14. ARN polymérase utilisable dans le procédé de l'une quelconque des revendications précédentes, capable de transcrire, sous la dépendance d'un promoteur, une cible polynucléotidique d'intérêt de séquence quelconque contenue dans une matrice polynucléotidique, en synthétisant, en présence de ladite matrice et en l'absence de facteur protéique associé, un produit de transcription contenant une séquence d'ARN complémentaire de ladite séquence, ladite ARN polymérase étant mutée et capable de synthétiser ledit produit de transcription avec un meilleur rendement lorsque ladite séquence cible de ladite matrice est constituée d'ARN que lorsqu'elle est constituée d'ADN.

15. ARN polymérase selon la revendication précédente, dans laquelle le rapport du rendement en produit de transcription de la matrice ARN au rendement en produit de transcription de la matrice ADN est supérieur à 2, et en particulier supérieur à 10.

16. ARN polymérase selon l'une quelconque des revendications 14 et 15, **caractérisée par le fait qu'**elle dérive par mutation d'une ARN polymérase de virus ou de phage.

17. ARN polymérase selon la revendication 16, **caractérisée par le fait que** ledit phage est un phage de E. Coli.

18. ARN polymérase selon l'une quelconque des revendications 14 à 17, **caractérisée par le fait qu'**elle possède une homologie de séquence protéique supérieure à 50 % en particulier supérieure à 80%, avec une ARN polymérase de type sauvage de la famille des ARN polymérases ADN-dépendantes incluant la T7 ARN polymérase, la T3 ARN polymérase et la SP6 ARN polymérase.

19. ARN polymérase selon la revendication 18, **caractérisée par le fait qu'**elle contient au moins une mutation dans une région correspondant à la séquence d'acides aminés 625-652 de la T7 ARN polymérase.

20. ARN polymérase selon la revendication 19, **caractérisée par le fait qu'**elle a la composition d'une ARN polymérase ADN-dépendante de type sauvage, à l'exception du fait qu'elle comporte au moins une mutation dans ladite région.

21. ARN polymérase selon la revendication 19 ou 20, **caractérisée par le fait qu'**elle comporte au moins une mutation en une position correspondant à l'une des positions 627, 628, 631, et 639 de la séquence d'acides aminés de la T7 ARN polymérase.

22. ARN polymérase selon l'une quelconque des revendications 19 à 21, **caractérisée par le fait que** ladite mutation comprend le remplacement d'un résidu d'acide aminé, choisi parmi l'arginine, la lysine, la sérine et la tyrosine, de l'ARN polymérase de type sauvage, par un autre résidu d'acide aminé.

23. ARN polymérase selon la revendication 22, **caractérisée par le fait que** ledit acide aminé remplacé est une arginine ou une lysine et/ou **par le fait que** ledit autre résidu d'acide aminé est un résidu d'alanine, de valine, de leucine, d'isoleucine, de glycine, de thréonine ou de sérine.

24. ARN polymérase selon l'une quelconque des revendications 19 à 23, **caractérisée par le fait que** ladite mutation comprend le remplacement de tout ou partie de ladite région par une région homologue présente dans une polymérase ARN-dépendante de type sauvage.

25. Gène codant pour une ARN polymérase telle que définie dans l'une quelconque des revendications 14 à 24.

26. Vecteur d'expression dans lequel est inséré un gène tel que défini dans la revendication précédente, ledit vecteur étant capable d'exprimer ladite ARN polymérase dans une cellule- hôte.

27. Cellule-hôte contenant un vecteur d'expression tel que défini dans la revendication précédente.

28. Procédé d'obtention d'une ARN polymérase telle que définie dans l'une quelconque des revendications 14 à 24, **caractérisé par** le fait : a) que l'on obtient de façon connue un gène codant pour une ARN polymérase de type sauvage, b) que l'on effectue au moins une mutation sur ledit gène, c) que l'on insère le gène muté obtenu dans un vecteur d'expression, d) que l'on fait s'exprimer ledit vecteur dans une cellule-hôte pour obtenir une ARN polymérase mutée et e) que l'on sélectionne parmi les ARN polymérases mutées obtenues celles qui présentent les propriétés d'une ARN polymérase telle que définie dans l'une des revendications 14 ou 15.

29. Utilisation d'une ARN polymérase capable de transcrire une matrice d'ARN, sous la dépendance d'un promoteur, en l'absence de facteur protéique auxiliaire, dans un procédé de transcription d'un brin matrice comprenant une séquence cible d'ARN, dans laquelle ladite ARN polymérase est choisie parmi la T7 ARN polymérase la SP6 ARN polymérase et les ARN polymérases telles que définies dans l'une quelconque des revendications 14 à 24.

30. Utilisation d'une ARN polymérase capable de transcrire une matrice d'ARN, sous la dépendance d'un promoteur, en l'absence de facteur protéique auxiliaire, dans un procédé de transcription d'un brin matrice comprenant une séquence cible d'ARN, dans laquelle ledit brin matrice est constitué d'ARN au moins entre la position +5 et l'extrémité 5' de la cible, ladite ARN polymérase étant une ARN polymérase sauvage de virus ou de phage.

31. Utilisation selon la revendication précédente, dans laquelle ladite ARN polymérase est choisie parmi la T7-, la T3- et la SP6- ARN polymérase.

## Claims

1. Method of amplifying any RNA target sequence, by transcription under the control of a promoter, in an RNA sample comprising said target sequence, in which said sample is brought into contact:
- with a reagent capable of hybridizing with said RNA comprising said target sequence,
- and with an enzymatic system comprising an RNA-dependent RNA polymerase activity,
under conditions allowing... the hybridization of said reagent with said RNA comprising said target sequence and under conditions allowing the functioning of said RNA-dependent RNA polymerase activity; in which said reagent contains:
(i) a first nucleotide strand comprising: a) a first nucleotide segment capable of playing the role of sense strand of a promoter for said RNA polymerase activity and b), downstream of said first segment, a second nucleotide segment comprising a sequence capable of hybridizing with a region of said RNA, and
(ii) in the hybridized state on the first strand, a second nucleotide strand comprising a third nucleotide segment capable of hybridizing with said first segment so as to form with it a functional double-stranded promoter;
and in which said RNA polymerase activity is capable of transcribing an RNA template, in the presence of said reagent hybridized with said template, in the absence of associated protein factor and in the absence of a ligase activity.

2. Method according to Claim 1, in which said third segment is flanked, at its upstream end, by a fourth nucleotide segment which is shorter than said second segment of the first strand.

3. Method according to Claim 2, in which said fourth segment is capable of hybridizing with an opposite portion of said second segment.

4. Method according to either of Claims 2 and 3, in which said fourth segment of said second strand is chosen from those whose sequence facilitates the initiation of transcription for said RNA polymerase.

5. Method according to any one of Claims 2 to 4, in which said second segment of said first strand contains a number of nucleotides at least equal to the sum of the number of nucleotides of said fourth segment, if it is present, and of the number of nucleotides of said sequence of the second segment which is capable of hybridizing with said region of said RNA.

6. Method according to any one of the preceding claims, in which said first and third segments consist of DNA.

7. Method according to any one of the preceding claims, in which said fourth segment consists of DNA.

8. Method according to any one of the preceding claims, in which said RNA polymerase is a virus or phage wild-type RNA polymerase.

9. Method according to Claim 8, in which said RNA polymerase is chosen from the family of RNA polymerases including the T7 RNA polymerase, T3 RNA polymerase and SP6 RNA polymerase.

10. Method according to Claim 8, in which said RNA polymerase is derived by mutation from an RNA polymerase chosen from the family of RNA polymerases including the T7, T3 and SP6 RNA polymerases.

11. Method according to Claim 10, in which said RNA polymerase contains at least one mutation in the region corresponding to the T7 RNA polymerase sequence containing amino acids 625 to 652.

12. Method according to Claim 11, in which said RNA polymerase is capable of transcribing a polynucleotide target sequence with a better yield when said target sequence consists of RNA than when it consists of DNA.

13. Method according to any one of the preceding claims, in which said enzyme system contains only an RNA polymerase activity.

14. RNA polymerase which can be used in the method of any one of the preceding claims, capable of transcribing, under the control of a promoter, a polynucleotide target of interest of any sequence contained in a polynucleotide template, by synthesizing, in the presence of said template and in the absence of associated protein factor, a product of transcription containing an RNA sequence complementary to said sequence, said RNA polymerase being mutated and capable of synthesizing said product of transcription with a better yield when said target sequence of said template consists of RNA than when it consists of DNA.

15. RNA polymerase according to the preceding claim, in which the ratio of the yield of product of transcription of the RNA template to the yield of product of transcription of the DNA template is greater than 2 and in particular greater than 10.

16. RNA polymerase according to either of Claims 14 and 15, **characterized in that** it is derived by mutation from a virus or phage RNA polymerase.

17. RNA polymerase according to Claim 16, **characterized in that** said phage is an E. coli phage.

18. RNA polymerase according to any one of Claims 14 to 17, **characterized in that** it possesses a protein sequence homology greater than 50%, and in particular greater than 80%, with a wild-type RNA polymerase of the family of DNA-dependent RNA polymerases including the T7 RNA polymerase, T3 RNA polymerase and SP6 RNA polymerase.

19. RNA polymerase according to Claim 18, **characterized in that** it contains at least one mutation in a region corresponding to the T7 RNA polymerase sequence containing amino acids 625-652.

20. RNA polymerase according to Claim 19, **characterized in that** it has the composition of a wild-type DNA-dependent RNA polymerase, with the exception of the fact that it contains at least one mutation in said region.

21. RNA polymerase according to Claim 19 or 20, **characterized in that** it contains at least one mutation at a position corresponding to one of positions 627, 628, 631 and 639 of the T7 RNA polymerase amino acid sequence.

22. RNA polymerase according to any one of Claims 19 to 21, **characterized in that** said mutation comprises the replacement of an amino acid residue, chosen from arginine, lysine, serine and tyrosine, of the wild-type RNA polymerase, with another amino acid residue.

23. RNA polymerase according to Claim 22, **characterized in that** said amino acid replaced is an arginine or a lysine and/or **in that** said other amino acid residue is an alanine, valine, leucine, isoleucine, glycine, threonine or serine residue.

24. RNA polymerase according to any one of Claims 19 to 23, **characterized in that** said mutation comprises the replacement of all or part of said region with a homologous region present in a wild-type RNA-dependent polymerase.

25. Gene encoding an RNA polymerase as defined in any one of Claims 14 to 24.

26. Expression vector into which a gene as defined in the preceding claim is inserted, said vector being capable of expressing said RNA polymerase in a host cell.

27. Host cell containing an expression vector as defined in the preceding claim.

28. Method of producing an RNA polymerase as defined in any one of Claims 14 to 24, **characterized in that**:
a) a gene encoding a wild-type RNA polymerase is obtained in a known manner, b) at least one mutation is performed on said gene, c) the mutated gene obtained is inserted into an expression vector, d) said vector is expressed in a host cell in order to obtain a mutated RNA polymerase and e) among the mutated RNA polymerases obtained, those which exhibit the properties of an RNA polymerase as defined in either of Claims 14 and 15 are selected.

29. Use of an RNA polymerase capable of transcribing an RNA template, under the control of a promoter, in the absence of auxiliary protein factor, in a method of transcription of a template strand comprising an RNA target sequence, in which said RNA polymerase is chosen from the T7 RNA polymerase, the SP6 RNA polymerase and the RNA polymerases as defined in any one of Claims 14 to 24.

30. Use of an RNA polymerase capable of transcribing an RNA template, under the control of a promoter, in the absence of auxiliary protein factor, in a method of transcription of a template strand comprising an RNA target sequence, in which said template strand consists of RNA at least between position +5 and the 5' end of the target, said RNA polymerase being a virus or phage wild-type RNA polymerase.

31. Use according to the preceding claim, in which said RNA polymerase is chosen from T7, T3 and SP6 RNA polymerase.

## Patentansprüche

1. Amplifikationsverfahren einer RNA-Zielsequenz durch eine Promotor-abhängige Transkription in einer RNA-Probe, die die Zielsequenz einschließt, wobei die Probe in Kontakt kommt
- mit einem Reagens, das in der Lage ist, mit der RNA, die die Zielsequenz enthält, zu hybridisieren,
- und mit einem Enzymsystem, das eine RNA-abhängige RNA-Polymeraseaktivität umfasst,
unter Bedingungen, die die Hybridisierung des Reagens mit der RNA, die die Zielsequenz umfasst, erlauben, und unter Bedingungen, die das Funktionieren der RNA-abhängigen RNA-Polymeraseaktivität erlauben; wobei das Reagens enthält
(i) einen ersten Nukleotidstrang, umfassend: a) einen ersten Nukleotidabschnitt, der in der Lage ist, die Rolle des codierenden Strangs eines Promotors für die RNA-Polymeraseaktivität zu spielen und b) stromabwärts des ersten Abschnitts einen zweiten Nukleotidabschnitt, der eine Sequenz umfasst, die in der Lage ist, mit einer Region der RNA zu hybridisieren, und
(ii) auf dem ersten Strang im hybridisierten Zustand, einen zweiten Nukleotidstrang, der einen dritten Nukleotidabschnitt umfasst, der in der Lage ist, mit dem ersten Abschnitt so zu hybridisieren, dass mit ihm ein funktioneller doppelsträngiger Promotor gebildet wird;
und wobei die RNA-Polymeraseaktivität in der Lage ist, in Gegenwart des auf die Matrix hybridisierten Reagens, in Abwesenheit eines assoziierten Proteinfaktors und in Abwesenheit einer Ligaseaktivität eine RNA-Matrix zu transkribieren.

2. Verfahren nach Anspruch 1, wobei der dritte Abschnitt stromaufwärts von einem vierten Nukleotidabschnitt flankiert ist, der kürzer ist als der zweite Abschnitt des ersten Strangs.

3. Verfahren nach Anspruch 2, wobei der vierte Abschnitt mit einem gegenüberliegenden Teil des zweiten Abschnitts hybridisieren kann.

4. Verfahren nach einem der Ansprüche 2 und 3, wobei der vierte Abschnitt des zweiten Strangs unter denjenigen ausgewählt ist, deren Sequenz die Initiation der Transkription für die RNA-Polymerase erleichtert.

5. Verfahren nach einem der Ansprüche 2 bis 4, wobei der zweite Abschnitt des ersten Strangs eine Anzahl von Nukleotiden enthält, die wenigstens der Summe der Anzahl an Nukleotiden des vierten Abschnitts, sofern er anwesend ist, und der Anzahl von Nukleotiden der Sequenz des zweiten Abschnitts, die zur Hybridisierung mit der Region der RNA in der Lage ist, entspricht.

6. Verfahren nach einem der vorhergehenden Ansprüche, wobei der erste und dritte Abschnitt aus DNA bestehen.

7. Verfahren nach einem der vorhergehenden Ansprüche, wobei der vierte Abschnitt aus DNA besteht.

8. Verfahren nach einem der vorhergehenden Ansprüche, wobei die RNA-Polymerase eine Wildtyp-RNA-Polymerase aus Virus oder Phage ist.

9. Verfahren nach Anspruch 8, wobei die RNA-Polymerase aus der Familie der RNA-Polymerasen, einschließend der T7-RNA-Polymerase, der T3-RNA-Polymerase und der SP6-RNA-Polymerase, ausgewählt ist.

10. Verfahren nach Anspruch 8, wobei sich die RNA-Polymerase durch Mutation von einer RNA-Polymerase aus der Familie der RNA-Polymerasen, einschließend der T7-, der T3- und der SP6-RNA-Polymerasen, ableitet.

11. Verfahren nach Anspruch 10, wobei die RNA-Polymerase mindestens eine Mutation in der Region entsprechend der Aminosäuresequenz 625 bis 652 der T7-RNA-Polymerase enthält.

12. Verfahren nach Anspruch 11, wobei die RNA-Polymerase in der Lage ist, eine Polynukleotid-Zielsequenz mit einer besseren Ausbeute zu transkribieren, wenn die Zielsequenz aus RNA statt aus DNA besteht.

13. Verfahren nach einem der vorhergehenden Ansprüche, wobei das Enzymsystem ausschließlich eine RNA-Polymeraseaktivität enthält.

14. RNA-Polymerase, die bei dem Verfahren nach einem der vorhergehenden Ansprüche verwendbar ist, die in der Lage ist, eine interessierende Polynukleotid-Zielsequenz aus einer beliebigen, in einer Polynukleotidmatrix enthaltenen Sequenz unter der Abhängigkeit eines Promotors zu transkribieren, wobei in Gegenwart der Matrix und in Abwesenheit eines assoziierten Proteinfaktors ein Transkriptionsprodukt, das eine zu dieser Sequenz komplementäre RNA-Sequenz umfasst, synthetisiert wird, wobei die RNA-Polymerase mutiert ist und in der Lage ist, das Transkriptionsprodukt mit einer besseren Ausbeute zu synthetisieren, wenn die Zielsequenz der Matrix aus RNA statt aus DNA besteht.

15. RNA-Polymerase nach dem vorhergehenden Anspruch, wobei das Verhältnis der Ausbeute an Transkriptionsprodukt der RNA-Matrix und der Ausbeute an Transkriptionsprodukt der DNA-Matrix größer als 2 und insbesondere größer als 10 ist.

16. RNA-Polymerase nach einem der Ansprüche 14 bis 15, **dadurch gekennzeichnet, dass** sie sich durch Mutation von einer RNA-Polymerase aus Virus oder Phage ableitet.

17. RNA-Polymerase nach Anspruch 16, **dadurch gekennzeichnet, dass** der Phage ein Phage von *E. coli* ist.

18. RNA-Polymerase nach einem der Ansprüche 14 bis 17, **dadurch gekennzeichnet, dass** sie eine Proteinsequenzhomologie von über 50 %, insbesondere von über 80 % zu einer Wiltyp-RNA-Polymerase aus der Familie der DNA-abhängigen RNA-Polymerasen, einschließlich der T7-RNA-Polymerase, der T3-RNA-Polymerase und der SP6-RNA-Polymerase, aufweist.

19. RNA-Polymerase nach Anspruch 18, **dadurch gekennzeichnet, dass** sie mindestens eine Mutation in einer Region enthält, die der Aminosäuresequenz 625 bis 652 der T7-RNA-Polymerase entspricht.

20. RNA-Polymerase nach Anspruch 19, **dadurch gekennzeichnet, dass** sie die Zusammensetzung einer DNA-abhängigen Wildtyp-RNA-Polymerase aufweist, mit der Ausnahme, dass sie in dieser Region mindestens eine Mutation enthält.

21. RNA-Polymerase nach einem der Ansprüche 19 bis 20, **dadurch gekennzeichnet, dass** sie mindestens eine Mutation an einer Stelle umfasst, die einer der Stellen 627, 628, 631 und 639 der Aminosäuresequenz der T7-RNA-Polymerase entspricht.

22. RNA-Polymerase nach einem der Ansprüche 19 bis 21, **dadurch gekennzeichnet, dass** die Mutation den Ersatz eines Aminosäurerests, ausgewählt aus Arginin, Lysin, Serin und Tyrosin, der Wildtyp-RNA-Polymerase durch einen anderen Aminosäurerest umfasst.

23. RNA-Polymerase nach Anspruch 22, **dadurch gekennzeichnet, dass** die ersetzte Aminosäure Arginin oder Lysin ist und/oder dass der andere Aminosäurerest ein Alanin-, Valin-, Isoleucin-, Glycin-, Threonin- oder Serinrest ist.

24. RNA-Polymerase nach einem der Ansprüche 19 bis 23, **dadurch gekennzeichnet, dass** die Mutation den Ersatz der gesamten Region oder eines Teils der Region durch eine homologe Region, die in einer Wildtyp-RNA-abhängigen Polymerase vorhanden ist, umfasst.

25. Gen, das eine RNA-Polymerase, wie in einem der Ansprüche 14 bis 24 definiert, codiert.

26. Expressionsvektor, in den ein Gen, wie in dem vorhergehenden Anspruch definiert, inseriert ist, der zur Expression der RNA-Polymerase in einer Wirtszelle in der Lage ist.

27. Wirtszelle, die einen Expressionsvektor, wie in dem vorhergehenden Anspruch definiert, enthält.

28. Verfahren zum Erhalten einer RNA-Polymerase, wie in einem der Ansprüche 14 bis 24 definiert, **dadurch gekennzeichnet**: a) dass ein Gen, das eine Wildtyp-RNA-Polymerase codiert, auf bekannte Weise erhalten wird, b) dass mindestens eine Mutation auf dem Gen durchgeführt wird, c) dass das erhaltene mutierte Gen in einen Expressionsvektor inseriert wird, d) dass der Vektor in einer Wirtszelle exprimiert wird, um eine mutierte RNA-Polymerase zu erhalten und e) dass aus den mutierten RNA-Polymerasen diejenigen ausgewählt werden, die die Eigenschaften einer RNA-Polymerase, wie in einem der Ansprüche 14 bis 15 definiert, aufweisen.

29. Verwendung einer RNA-Polymerase, die in der Lage ist, eine RNA-Matrix unter der Abhängigkeit eines Promotors in Abwesenheit eines assoziierten Proteinfaktors durch ein Transkriptionsverfahren eines Matrixstrangs, der eine RNA-Zielsequenz umfasst, zu transkribieren, wobei die RNA-Polymerase aus der T7-RNA-Polymerase, der SP6-RNA-Polymerase und den RNA-Polymerasen nach einem der Ansprüche 14 bis 24 ausgewählt ist.

30. Verwendung einer RNA-Polymerase, die in der Lage ist, eine RNA-Matrix unter der Abhängigkeit eines Promotors in Abwesenheit eines assoziierten Proteinfaktors durch ein Transkriptionsverfahren eines Matrixstrangs, der eine RNA-Zielsequenz umfasst, zu transkribieren, wobei der Matrixstrang aus RNA mindestens zwischen der Stelle +5 und dem 5'-Ende der Zielsequenz besteht, wobei die RNA-Polymerase eine Wildtyp-RNA-Polymerase aus Virus oder Phage ist.

31. Verwendung nach dem vorhergehenden Anspruch, wobei die RNA-Polymerase aus den T7-, T3- und SP6-RNA-Polymerasen ausgewählt ist.
